(19) Europäisches Patentamt
European Patent Office
Office européen des brevets

(11) EP 0 538 297 B1

(12) EUROPEAN PATENT SPECIFICATION

(45) Date of publication and mention of the grant of the patent:
**19.09.2001 Bulletin 2001/38**

(21) Application number: **91912145.9**

(22) Date of filing: **28.06.1991**

(51) Int Cl.[7]: **C07D 487/06**, A61K 31/55, A61P 31/18 // (C07D487/06, 243:00, 235:00)

(86) International application number:
**PCT/EP91/01224**

(87) International publication number:
**WO 92/00979 (23.01.1992 Gazette 1992/03)**

(54) **ANTIVIRAL TETRAHYDROIMIDAZO 1,4]BENZODIAZEPIN-2-(THIO)ONES**

ANTIVIRALE TETRAHYDROIMIDAZO[1,4]BENZODIAZEPIN-2-(THIO)ONE

TETRAHYDROIMIDAZO 1,4]BENZODIAZEPINE-2-(THIO)ONES ANTIVIRALES

(84) Designated Contracting States:
**AT BE CH DE DK ES FR GB GR IT LI LU NL SE**

(30) Priority: **06.07.1990 US 549349**

(43) Date of publication of application:
**28.04.1993 Bulletin 1993/17**

(73) Proprietor: **JANSSEN PHARMACEUTICA N.V.
2340 Beerse (BE)**

(72) Inventors:
- **KUKLA, Michael, Joseph
Maple Glen, PA 19002 (US)**
- **BRESLIN, Henry, Joseph
Lansdale, PA 19446 (US)**
- **RAEYMAEKERS, Alfons, Herman, Margaretha
B-2340 Beerse (BE)**
- **VAN GELDER, Josephus, Ludovicus, Hubertus
B-2460 Kasterlee (BE)**
- **JANSSEN, Paul, Adriaan, Jan
B-2350 Vosselaar (BE)**

(56) References cited:
**EP-A- 0 336 466** **EP-A- 0 417 840**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 538 297 B1

## Description

Background of the invention

In the Eur. J. Med. Chem. 1978, 13, 53-59, there are described three tetrahydroimidazo-[4,5,1-jk][1,4]benzodiazepines. EP-A-0,336,466, published October 11, 1989 discloses antiviral tetrahydroimidazo[1,4]benzodiazepinones. EP-A-0,417.840, published March 20, 1991, discloses antiviral tetrahydroimidazo[1,4]benzodiazepines. In Nature 1990, 343, 470, there are described the same tetrahydroimidazo[1,4]benzodiazepinones and a few corresponding thiones.

Description of the invention

The present invention is concerned with tetrahydroimidazo[1,4]benzodiazepines having the formula:

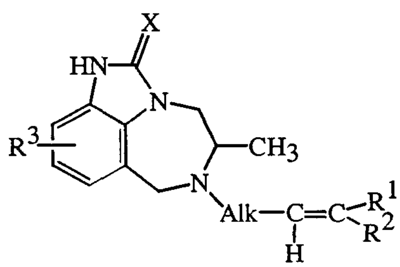

(I),

the pharmaceutically acceptable acid addition salts and the stereochemically isomeric forms thereof, wherein

X is O or S;
Alk is $C_{1-6}$alkanediyl;
$R^1$ and $R^2$ are $C_{1-4}$alkyl;
$R^3$ is methyl or chloro.

The compounds of formula (I) may also exist in their tautomeric form. Said tautomeric form, although not explicitly indicated in the above formula, is intended to be included within the scope of the present invention.

In the foregoing definitions the term halo is generic to fluoro, chloro, bromo and iodo; $C_{1-4}$alkyl defines straight and branched chain saturated hydrocarbon radicals having from 1 to 4 carbon atoms such as, for example, methyl, ethyl, propyl, 1-methylethyl, butyl, 1-methylpropyl, 2-methylpropyl, 1,1-dimethylethyl;
$C_{1-6}$alkanediyl defines bivalent straight or branched chain hydrocarbon radicals containing 1 to 6 carbon atoms such as, for example, 1,2-ethanediyl, 1,3-propanediyl, 1,4-butanediyl, 1,5-pentanediyl, 1,6-hexanediyl and the branched isomers thereof.

Depending on the nature of the various substituents, the compounds of formula (I) may have several asymmetric carbon atoms . Unless otherwise mentioned or indicated, the chemical designation of compounds denotes the mixture of all possible stereochemically isomeric forms, said mixtures containing all diastereomers and enantiomers of the basic molecular structure. The absolute configuration of each chiral center may be indicated by the stereochemical descriptors R and S. Stereochemically isomeric forms of the compounds of formula (I) are obviously intended to be embraced within the scope of the invention.

Pure stereochemically isomeric forms of the compounds of formula (I) may be obtained by the application of art-known procedures. Diastereoisomers may be separated by physical separation methods such as selective crystallization and chromatographic techniques, e.g., counter current distribution, liquid chromatography ; and enantiomers may be separated from each other by the selective crystallization of their diastereomeric salts with optically active acids. Pure stereochemically isomeric forms may also be derived from the corresponding pure stereochemically isomeric forms of the appropriate starting materials, provided that the reaction occurs stereospecifically.

The compounds of formula (I) have basic properties and, consequently, they may be converted to their therapeutically active non-toxic acid addition salt forms by treatment with appropriate acids, such as, for example, inorganic acids, e.g. hydrochloric acid, hydro-bromic acid, sulfuric acid, nitric acid, phosphoric acid; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic and 4-amino-2-hydroxybenzoic acid. Conversely the salt form can be converted by treatment with

alkali into the free base form. The term pharmaceutically acceptable acid addition salts also comprises the solvates which the compounds of formula (I) may form and said solvates are intended to be included within the scope of the present invention. Examples of such solvates are e.g. the hydrates and, alcoholates.

A first particular subgroup comprises these compounds wherein X represents O.

A second particular subgroup comprises these compounds wherein X represents S.

Particular compounds within the above-mentioned subgroups are these compounds wherein the carbon atom bearing the $CH_3$ substituent has the (S)-configuration.

More particular compounds are those particular compounds wherein $R^1$ and $R^2$ each independently represent $C_{1-3}$alkyl.

The most interesting compound is (+)-(S)-8-chloro-6-(3-ethyl-2-pentenyl)-4,5,6,7-tetrahydro-5-methylimidazo [4,5,1-jk][1,4]benzodiazepin-2(1H)-thione.

The compounds of formula (I) can generally be prepared by condensing a 9-amino-2,3,4,5-tetrahydro-1H-1,4-benzodiazepine of formula (II) with a reagent of formula (III), wherein L is an appropriate leaving group, such as, for example, halo, e.g. chloro or bromo.

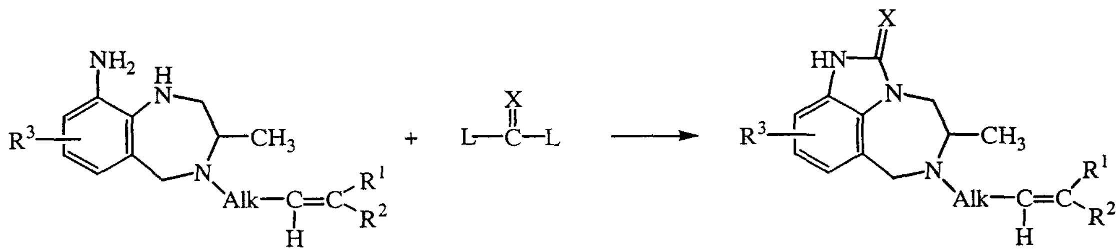

(II) (III) (I)

Appropriate agents of formula (III) are for example urea, di($C_{1-6}$alkyl)-carbonate, carbonoic dichloride, trichloromethyl chloroformate, 1,1'-carbonylbis[1H-imidazole], alkali metal, alkaline earth metal or ammonium isocyanates, phenyl isocyanate, benzoyl isocyanate, thiourea, carbonothioic dichloride, carbon disulfide, 1,1'-carbonothioyl-bis[1H-imidazole], xanthogenates, alkali metal, alkaline earth metal or ammonium isothiocyanates, phenyl isothiocyanate, benzoyl isothiocyanate and 1,3-dithiolane-2-thione. Said condensation reaction may conveniently be conducted by stirring and optionally heating the reactants in a reaction-inert solvent, such as, for example, an aromatic hydrocarbon, e.g. benzene, methylbenzene and dimethylbenzene; a halogenated hydrocarbon, e.g. trichloromethane, tetrachloromethane, chlorobenzene; an ether, e.g. tetrahydrofuran, 1,4-dioxane, 1,1'-oxybisbutane, 1,1'-oxybis(2-methoxyethane), 1,2-bis(2-methoxyethoxy)ethane; a dipolar aprotic solvent, e.g. N,N-dimethylformamide, N,N-dimethylacetamide, dimethyl sulfoxide, 1-methyl-2-pyrrolidinone, pyridine, methylpyridine, dimethylpyridine and, tetrahydrothiophene 1,1-dioxide; or a mixture of such solvents. In some instances however, it may be preferable to heat the reactants without a solvent. Further it may be appropriate to add to the reaction mixture a base such as, for example, a tertiary amine, e.g. N,N-diethylethanamine, N-ethyl-N-(1-methylethyl)-2-propanamine and, 4-methylmorpholine. When said reagent of formula (III) is carbon disulfide, the reaction can also be conducted conveniently in an alkanol such as, for example, methanol, ethanol and propanol in the presence of a base such as sodium or potassium hydroxide or in carbon disulfide as solvent and in the presence of a suitable base such as, for example, an alkyl magnesium halide, e.g. ethyl magnesium bromide, an alkyl lithium, e.g. butyllithium, an amine, e.g., N,N-diethylethanamine, a carbodiimide, e.g. N,N-dicyclohexylcarbodiimide. Or, alternatively the latter reaction may also be conducted in basic solvent such as, for example, pyridine, in the presence of a phosphite such as, for example, diphenylphosphite.

The compounds of formula (I) can also be prepared by reacting a 4,5,6,7-tetrahydroimidazo[4,5,1-jk][1,4] benzodiazepine derivative of formula (IV) with a reagent of formula $M_2X$ (V), wherein X is as defined hereinabove.

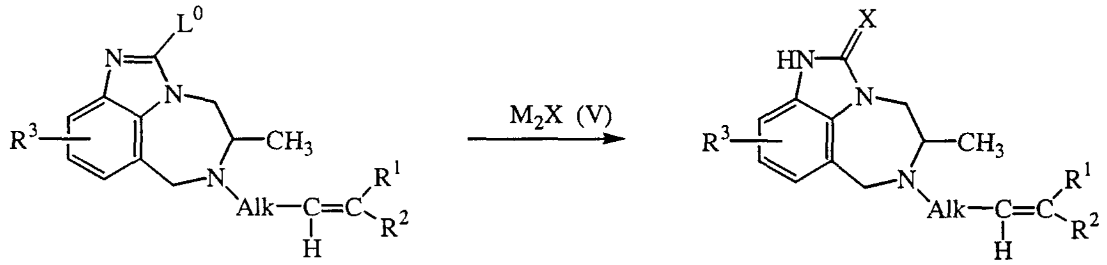


(IV) (I)

In formula (IV), $L^0$ is a reactive leaving group such as, for example, halo, e.g. chloro, bromo. Appropriate reagents of formula $M_2X$ (V) are for example, water, urea, thiourea, an alkali metal thiosulfate, e.g. sodium thiosulfate. Said reaction can conveniently be conducted by stirring and optionally heating the reactants in a reaction-inert solvent such as, for example, water, an alkanol, e.g., methanol, ethanol, 1-propanol, 2-propanol, butanol, 1,2-ethanediol; or an aromatic hydrocarbon, e.g. benzene, methylbenzene, dimethylbenzene; a halogenated hydrocarbon, e.g. trichloromethane, tetrachloromethane, chlorobenzene; an ether, e.g. tetrahydrofuran, 1,4-dioxane, 1,1'-oxybisbutane, 1,1'-oxybis(2-methoxyethane), 1,2-bis(2-methoxyethoxy)ethane; a dipolar aprotic solvent, e.g. N,N-dimethylformamide, N,N-dimethylacetamide, dimethyl sulfoxide, 1-methyl-2-pyrrolidinone, pyridine, methylpyridine, dimethylpyridine, tetrahydrothiophene 1,1-dioxide; or a mixture of such solvents. In some cases it may be appropriate to conduct said reaction in an excess of the reagent of formula (V), optionally in the presence of a reaction-inert solvent as defined above. In particular, the reaction may be conducted at an elevated temperature, more particularly the reflux temperature of the reaction mixture. Further, it may be appropriate to add to the reaction mixture a base such as, for example, an amine, e.g. N,N-diethylethanamine, N-ethyl-N-(1-methylethyl)-2-propanamine, 4-methylmorpholine.

The compounds of formula (I) may also be obtained by N-alkylating an intermediate of formula (VI) with a reagent of formula $R^1R^2C=CH\text{-}Alk\text{-}W$ (VII) wherein W represents an appropriate reactive leaving group such as, for example, halo, e.g. chloro, bromo or iodo; or a sulfonyloxy group, e.g. benzenesulfonyloxy, 4-methylbenzenesulfonyloxy, methanesulfonyloxy.

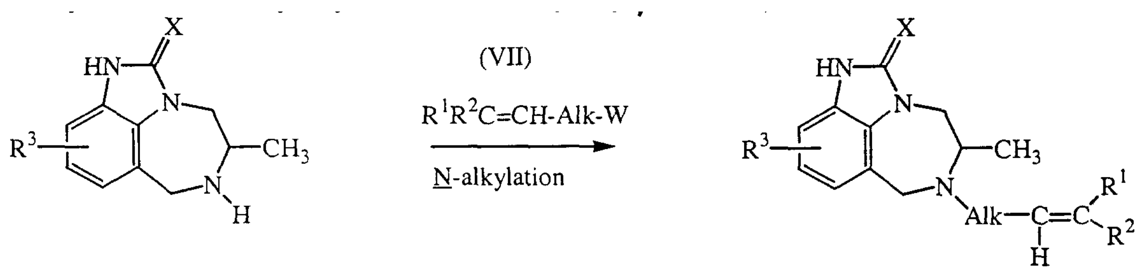


(VI) (I)

Said N-alkylation reaction may conveniently be conducted in a reaction-inert solvent such as, for example, an aromatic hydrocarbon, e.g., benzene, methylbenzene, dimethylbenzene; a lower alkanol, e.g., methanol, ethanol, 1-butanol; a ketone, e.g., 2-propanone, 4-methyl-2-pentanone; an ether, e.g., 1,4-dioxane, 1,1'-oxybisethane, tetrahydrofuran and the like; a dipolar aprotic solvent, e.g. N,N-dimethylformamide, N,N-dimethylacetamide, nitrobenzene, dimethyl sulfoxide, 1-methyl-2-pyrrolidinone, or a mixture of such solvents. The addition of an appropriate base such as, for example, an alkali metal carbonate or hydrogen carbonate, e.g. sodium carbonate, sodium hydrogen carbonate ; sodium hydride or an organic base such as, for example, N,N-diethylethanamine or N-(1-methylethyl)-2-propanamine may be utilized to pick up the acid which is liberated during the course of the reaction. In some circumstances the addition of an iodide salt, preferably an alkali metal iodide, e.g. potassium iodide, is appropriate. Somewhat elevated temperatures and stirring may enhance the rate of the reaction.

The compounds of formula (I) wherein X is S, said compounds being represented by formula (I-b-2), can be prepared by thionation of the compounds of formula (I) wherein X is O, said compounds represented by formula (I-b-1), with 2,4-bis(4-methoxyphenyl)-1,3-dithia-2,4-diphosphetane-2,4-disulfide (Lawesson's reagent) in an appropriate reaction-inert solvent. Such solvents are for example, aromatic hydrocarbons, e.g. benzene, methylbenzene, dimethylbenzene, dipolar aprotic solvents, e.g. hexamethylphosphoric triamide (HMPA).

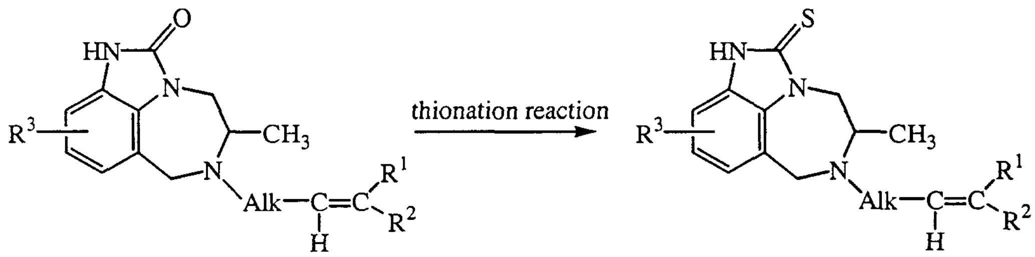


(I-b-1) (I-b-2)

Alternatively, the compounds of formula (I-b-2) may also be obtained by thionation of the compounds of formula (I-b-1) with phosphorus pentasulfide.

The compounds of formula (I-b-2) may also be obtained by direct thiation of a tetrahydroimidazo[4,5,1-jk][1,4] benzodiazepine of formula (IX) with elemental sulfur at an elevated temperature.

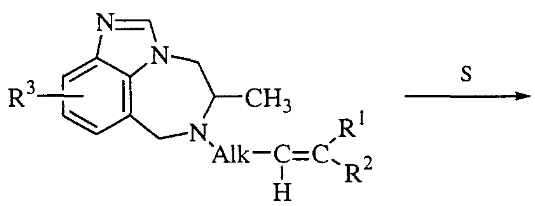


(IX) (I-b-2)

Said reaction may conveniently be conducted without a solvent at a temperature above 200°C, more particularly a temperature in the range of 230 to 250°C.

The compounds of formula (I-b-2) may alternatively be prepared by the combined reduction-thiocarbonylation of a 9-nitrobenzodiazepine of formula (X) in the presence of an alkali metal sulfide or hydrogen sulfide, and carbon disulfide.

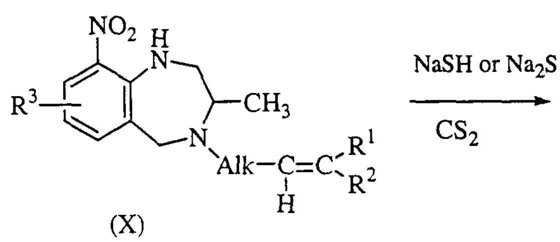


(X) (I-b-2)

Said reduction-thiocarbonylation reaction may conveniently be conducted by stirring the reactants in a reaction-inert solvent, optionally at an elevated temperature.

The compounds of formula (I) may also be prepared by cyclizing a benzimidazole of formula (XI) in a suitable reaction-inert solvent, optionally in the presence of a base and optionally at an elevated temperature.

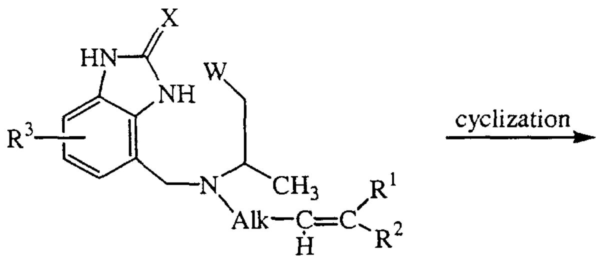


(I)

(XI)

In formula (XI), W represents a reactive leaving group as defined hereinbefore. Said cyclization reaction may conveniently be conducted by stirring, and, if desired, heating the starting material. Suitable solvents are, for example, aromatic hydrocarbons, e.g. benzene, methylbenzene, dimethylbenzene, halogenated hydrocarbons, e.g. trichloromethane, tetrachloromethane, chlorobenzene, ethers, e.g. tetrahydrofuran, 1,4-dioxane, dipolar aprotic solvents e. g. N,N-dimethyl-formamide, N,N-dimethylacetamide, acetonitrile, dimethylsulfoxide, pyridine. Bases which may conveniently be employed in said cyclization reaction are, for example, alkali metal or alkaline earth metal carbonates, hydrogen carbonates, hydroxides, oxides, amides, hydrides. In some instances the addition to the reaction mixture of a iodide salt, preferably an alkali metal iodide, e.g. potassium iodide, may be advantageous.

In all of the foregoing and in the following preparations, the reaction products may be isolated from the reaction mixture and, if necessary, further purified according to methodologies generally known in the art.

A number of intermediates and starting materials in the foregoing preparations are known compounds which may be prepared according to art-known methodologies of preparing said or similar compounds and some intermediates are new. A number of such preparation methods will be described hereinafter in more detail.

The intermediates of formula (II) can generally be prepared from a 9-aminobenzodiazepine of formula (II-a) following N-alkylation reaction procedures such as described hereinabove for the preparation of the compounds of formula (I) from an intermediate of formula (VI) with an alkylating reagent (VII).

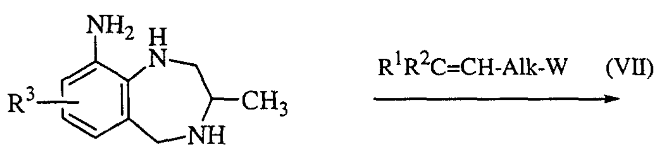


(II)

(II-a)

In order to simplify the following reaction schemes, the N-alkylated and the $N^4$-unsubstituted intermediates (wherein $R^1R^2C$=CH-Alk- is replaced by hydrogen) will be represented hereinafter by formulae wherein $N^4$ is substituted with $R^{1H}$, said $R^{1H}$ defining $R^1R^2C$=CH-Alk- and hydrogen.

In all of the following reaction schemes, the intermediates wherein $R^{1H}$ is hydrogen can be converted into intermediates wherein $R^{1H}$ is $R^1R^2C$=CH-Alk- following the above described N-alkylation procedures with an alkylating reagent of formula $R^1R^2C$=CH-Alk-W (VII)

The intermediates of formula (II-H), said intermediates representing the intermediates of formula (II) and (II-a) can generally be prepared following the reaction steps shown in the reaction scheme 1 below.

Scheme 1

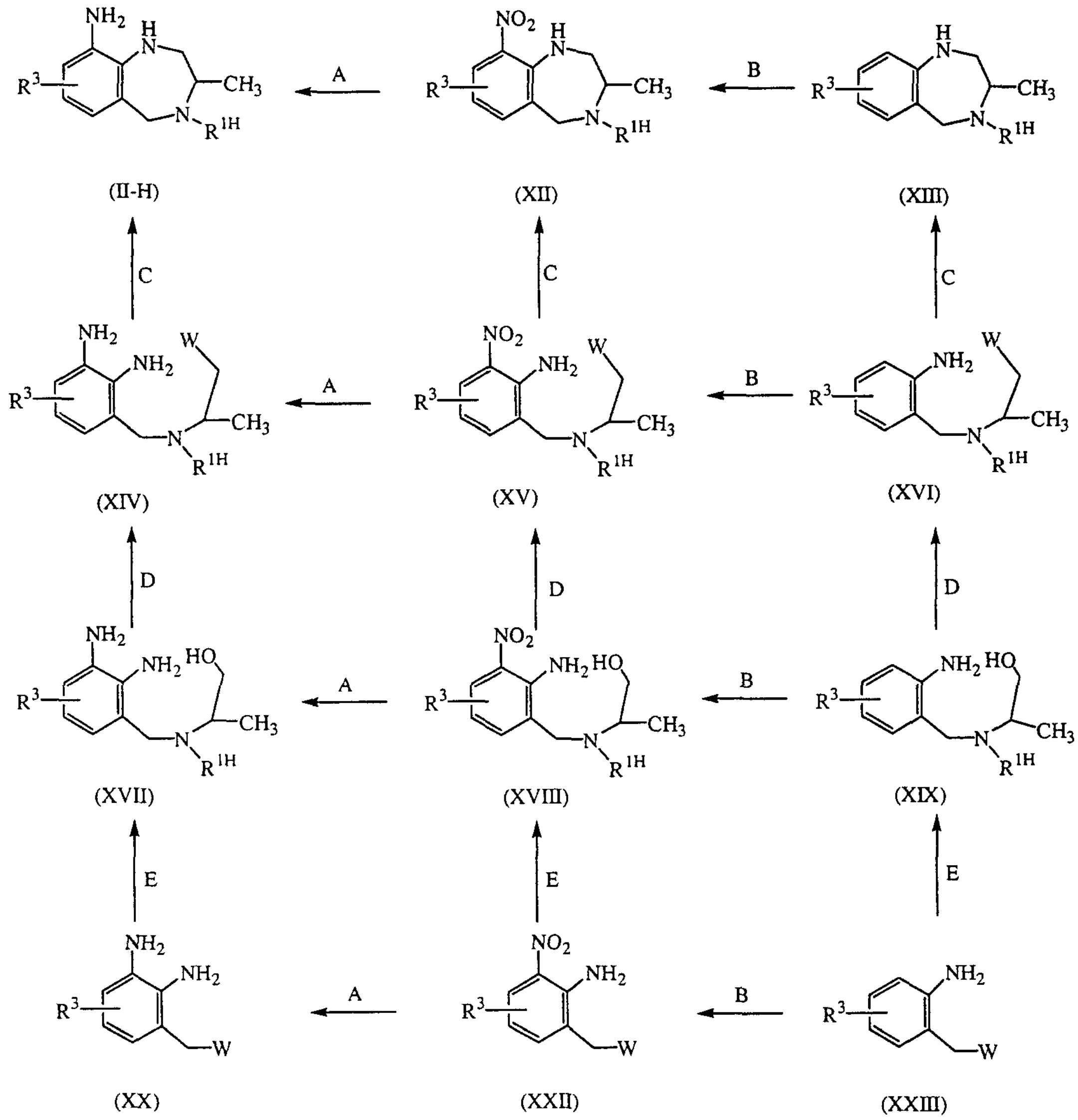


(II-H) (XII) (XIII)

(XIV) (XV) (XVI)

(XVII) (XVIII) (XIX)

(XX) (XXII) (XXIII)

**A :** nitro-to-amine reduction
**B :** nitration
**C :** cyclization
**D :** -OH-to-W activation
**E :** N-alkylation : $R^{1H}NH-CH(CH_3)-CH_2OH$ (XXI)

The aniline derivatives in the above reaction scheme may conveniently be prepared by reduction of the corresponding nitrobenzene derivatives following art-known nitro-to-amine reduction procedures (reaction step A). Said reduction may conveniently be conducted by treatment of said nitrobenzenes with a reducing agent such as, for example, a complex metal hydride, e.g. lithium aluminum hydride; a hydride, e.g. diborane, aluminum hydride, in a reaction-inert solvent such as, for example, 1,1'-oxybisethane, tetrahydrofuran, 1,4-dioxane, 1,2-dimethoxyethane, optionally in the presence of a cosolvent such as an aromatic hydrocarbon, e.g. benzene, methylbenzene, and, if desired, at an elevated temperature. Alternatively, said reduction may also be accomplished by treatment of said nitrobenzene derivatives with sodium dithionite, sodium sulfide, sodium hydrogen sulfide, titanium(III) chloride in a suitable solvent, in particular water.

Said nitro-to-amine reduction may also be conducted following art-known catalytic hydrogenation procedures. For example, said reduction may be carried out by stirring the reactants under a hydrogen atmosphere and in the

presence of an appropriate catalyst such as, for example, palladium-on-charcoal, platinum-on-charcoal, Raney-nickel. Suitable solvents are, for example, water, alkanols, e.g. methanol, ethanol, esters, e.g. ethyl acetate. In order to enhance the rate of said reduction reaction it may be advantageous to elevate the temperature and/or the pressure of the reaction mixture. Undesired further hydrogenation of certain functional groups in the reactants and the reaction products may be prevented by the addition of a catalyst poison such as, for example, thiophene, to the reaction mixture.

The nitrobenzene derivatives in the above reaction scheme 1 can be prepared from benzenamine derivatives following art-known nitration procedures (reaction step B). For example, the starting materials may be nitrated by treatment with concentrated or fuming nitric acid in the presence of concentrated sulfuric acid and optionally in the presence of a cosolvent such as, for example, a halogenated hydrocarbon, e.g. dichloromethane, trichloromethane, tetrachloromethane. Alternatively, said nitration may in some instances also be accomplished by adding the nitrate salt of the starting material to concentrated sulfuric acid.

The benzodiazepine derivatives (II-H), (XII) and (XIII) may be obtained from the corresponding aniline derivatives (XIV), (XV) and (XVI) (Reaction step C) following the cyclization procedures such as described hereinabove for the preparation of the compounds of formula (I) from intermediates of formula (XI).

Said aniline derivatives in turn, wherein W is a reactive leaving group as defined hereinbefore, may be prepared from the corresponding alkanols by treatment with a halogenating reagent such as, for example, thionyl chloride, phosphoryl chloride, phosphorous trichloride; or by treatment with a sulfonylating reagent, e.g. methanesulfonyl chloride, 4-methylbenzenesulfonyl chloride (Reaction step D).

Said alkanols may be prepared by N-alkylating appropriately substituted benzene derivatives of formulae (XX), (XXII) or (XXIII) with an aminoethanol derivative of formula $R^{1H}NH-CH(CH_3)-CH_2OH$ (XXI) following art-known N-alkylation procedures such as described hereinabove (Reaction step E).

The intermediates of formula (II-H) may also be obtained following the reaction steps shown in reaction scheme 2 below. Reaction steps designated A through D are intended to refer back to the analogous reaction steps described in the previous reaction scheme.

For example, the intermediates of formula (II-H) can also be prepared from an 9-amino- or a 9-nitrobenzodiazepin-5-one of formula (XXIV) or (XXV) by reduction with a complex metal hydride e.g. lithium aluminum hydride in a suitable reaction-inert solvent such as, for example, 1,2-dimethoxyethane, 1,1'-oxybis(2-methoxyethane), 2,5,8,11-tetraoxadodecane, methoxybenzene (Reaction steps F and G). In order to enhance the rate of said reduction reaction it may be advantageous to employ an excess of the reducing reagent and to conduct said reaction at an enhanced temperature of the reaction mixture, in particular the reflux temperature of the reaction mixture.

The intermediates of formula (XXV) can alternatively be obtained from an appropriately substituted nitrobenzene (XXXVI) by a condensation reaction (reaction step H) with a diamino reagent $R^{1H}NH-CH(CH_3)-CH_2-NH_2$ of formula (XXXVII) in a suitable reaction-inert solvent such as, for example, an alkanol, e.g. methanol, ethanol, 2-propanol, 1-butanol; an aromatic hydrocarbon, e.g. benzene, methylbenzene, dimethylbenzene; a halogenated hydrocarbon, e.g. trichloromethane, tetrachloromethane; an ether, e.g. tetrahydofuran, 1,4-dioxane, 1,1'-oxybisbutane, 1,1'-oxybis (2-methoxyethane); a ketone, e.g. 2-propanone, 4-methyl-2-pentanone; a dipolar aprotic solvent, e.g. N,N-dimethylformamide, N,N-dimethylacetamide, dimethyl sulfoxide; or a mixture of such solvents. It may be appropriate to add a base such as an alkali metal or an alkaline earth metal carbonate, e.g. sodium carbonate, sodium hydrogen carbonate, to the reaction mixture. Said condensation reaction can conveniently be conducted at an elevated temperature, in particular at the reflux temperature of the reaction mixture.

Scheme 2

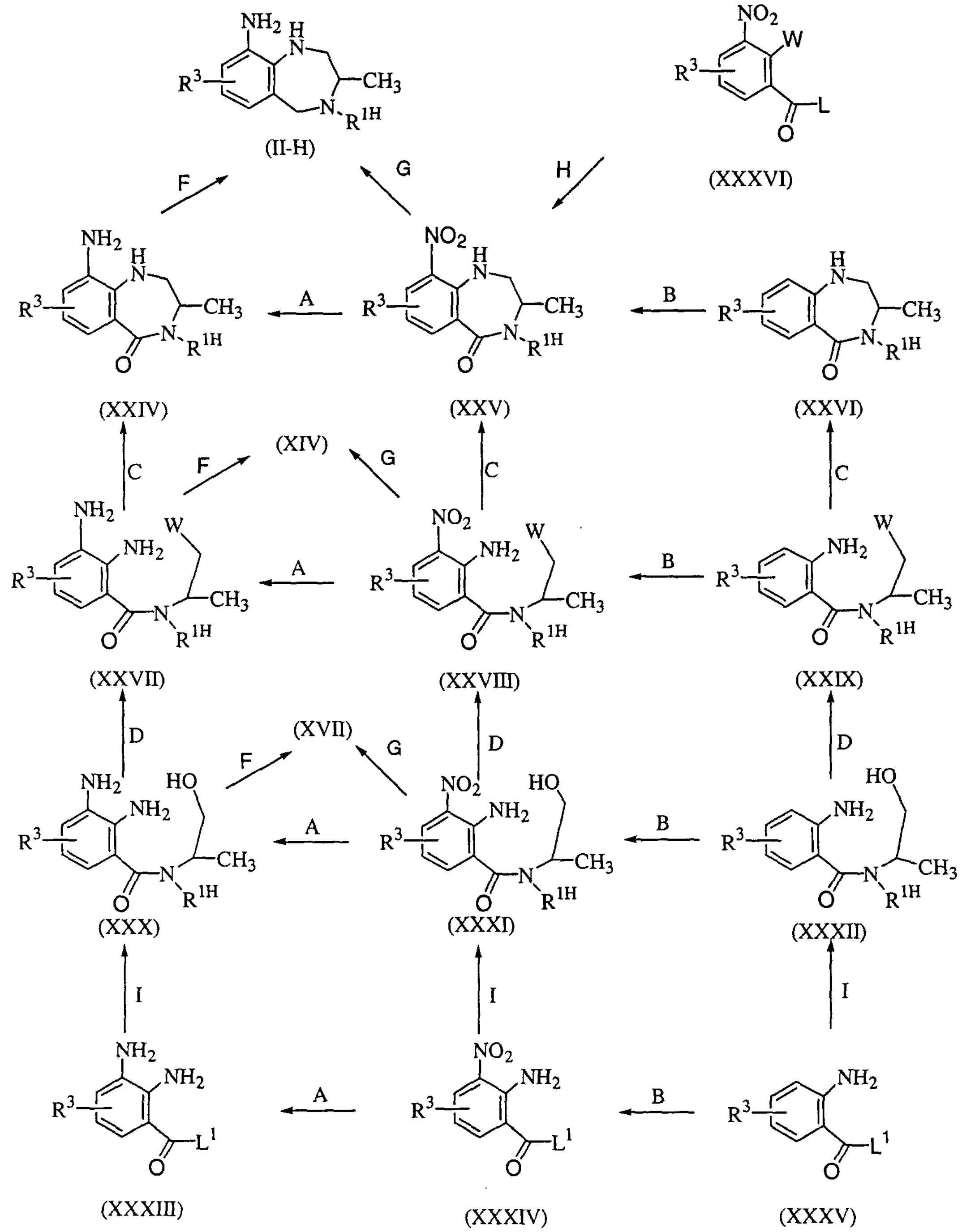


**F** : amide-to-amine reduction
**G :** (nitro-to-amino) and (amide-to-amine) reduction
**H :** cyclization; $R^{1H}$-NH-CH($CH_3$)-$CH_2$-$NH_2$ (XXXVII)
**I:** N-acylation reaction; $R^{1H}$NH-CH($CH_3$)-$CH_2$OH (XXI)

The amide derivatives (XXX), (XXXI) and (XXXII) in the above reaction scheme can conveniently be prepared by N-acylating an ethanolamine of formula $R^{1H}$NH-CH($CH_3$)-$CH_2$-OH (XXI) with an appropriately substituted 2-aminobenzoic acid derivative of formula (XXXIII), (XXXIV) or (XXXV) wherein $L^1$ represents hydroxy or a leaving group such as, for example, halo, e.g. chloro or bromo, alkylcarbonyloxy, e.g. acetyl, alkyloxy, e.g. methoxy, ethoxy, or imidazolyl. Said N-acylation reaction (reaction step I) may be carried out by stirring the reactants in a reaction-inert solvent,

optionally at an elevated temperature. In those instances where $L^1$ represents hydroxy, said N-acylation reaction may also be carried out by treating the reactants with reagents capable of forming amides such as, for example, N,N-dicyclohexylcarbodiimide (DCC) optionally in the presence of a catalyst such as hydroxybenzotriazole (HOBT) or 4-dimethylaminopyridine (DMAP); 2-chloro- 1-methylpyridinium iodide, 1,1'-carbonylbis[1H-imidazole], 1,1'-sulfonylbis [1H-imidazole]. Suitable solvents are halogenated hydrocarbons, e.g. dichloromethane, trichloromethane and the like, ethers, e.g. tetrahydrofuran, 1,4-dioxane, dipolar aprotic solvents, e.g. N,N-dimethylformamide, N,N-dimethylacetamide, pyridine; or mixtures of such solvents.

The intermediates of formula (II-H) can also be prepared from a benzodiazepinedione of formula

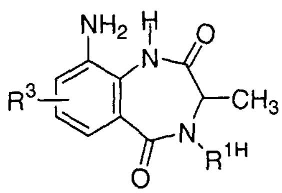

(XXXVIII)

following the reduction procedures as described hereinabove for converting intermediates (XXIV) or (XXV) into intermediate (II-H). The preparation of the intermediates of formula (XXXVIII) may generally be conducted following the reaction pathways described in scheme 3 hereinbelow.

Scheme 3

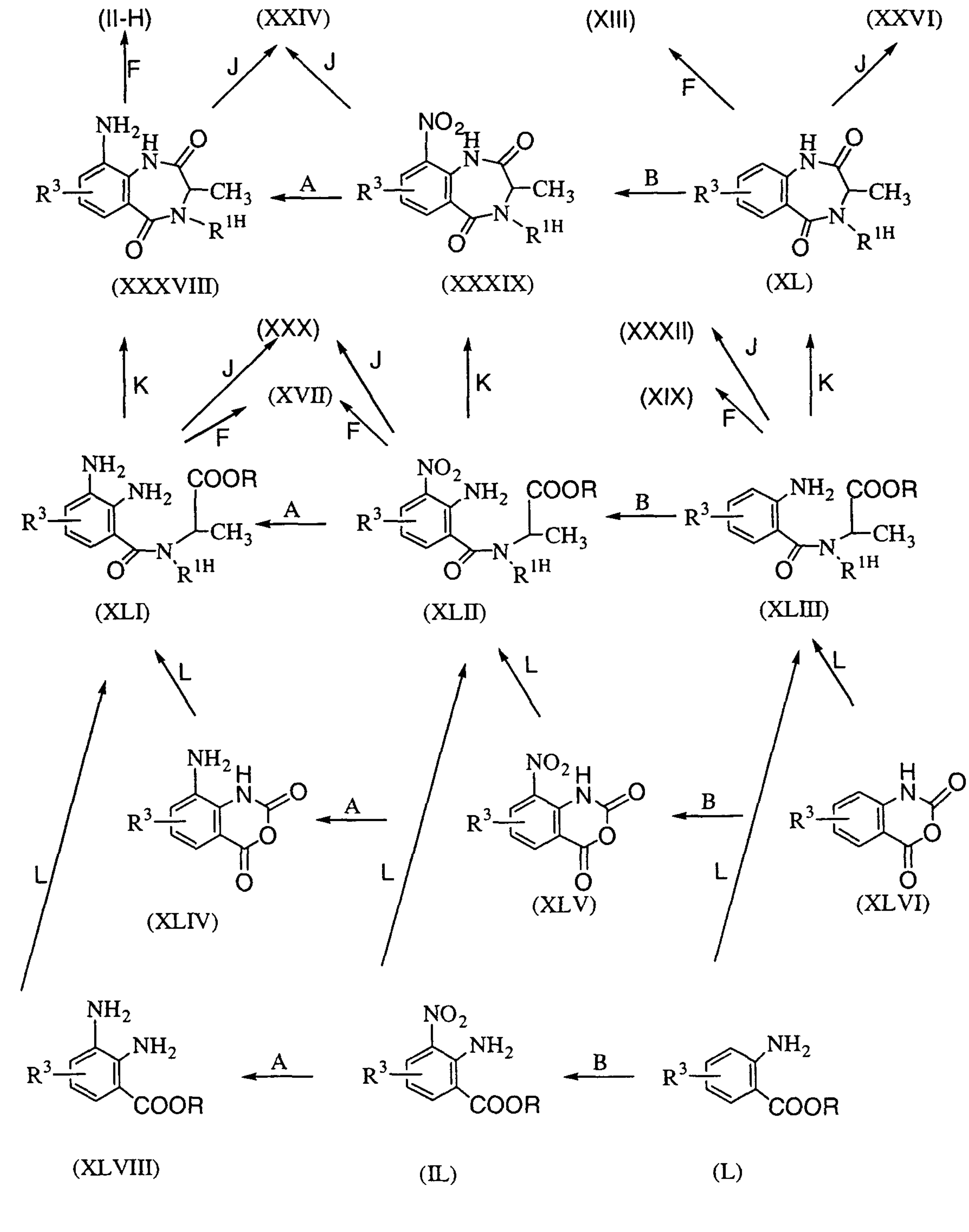


**J :** (nitro-to-amino) and/or aliphatic amide-to-amine reduction

**K :** cyclization to benzodiazepinedione

**L** : N-acylation of $R^{1H}NH-CH(CH_3)-COOR$ (XLVII)

In a number of the intermediates shown in scheme 3, for example in (XXXVIII), (XXXIX), (XL), (XLI), (XLII) and (XLIII) it is further possible to selectively reduce functional groups such as the nitro group, the ester group and/or the aliphatic amide group, in the presence of the aromatic amide group (reaction step J). Said selective reduction may

be carried out by treatment of the appropriate starting material with a complex metal hydride such as, for example, lithium aluminum hydride in a reaction-inert solvent such as, for example, tetrahydrofuran, 1,4-dioxane.
Alternatively, said selective reduction may also be performed by treatment of the appropriate starting material with sodium bis(2-methoxyethoxy) aluminum hydride, or with sodium borohydride in the presence of a suitable metal salt such as, for example, calcium chloride, cerium(III) chloride, aluminum chloride, zirconium(IV) chloride, in a reaction-inert solvent, in particular an ether.

The benzodiazepinediones in scheme 3 can be obtained by cyclizing (reaction step K) the corresponding acyclic intermediates of formula (XLI), (XLII) and (XLIII), wherein R represents a group such as $C_{1-6}$alkyl or aryl,

a) by heating without a solvent under an inert atmosphere, optionally under reduced pressure;
b) by treating with a bifunctional catalyst such as, for example, acetic acid, 2-hydroxypyridine, pyrazole, 1,2,4-triazole, in a reaction-inert solvent such as, for example, an aromatic hydrocarbon, e.g. methylbenzene, dimethylbenzene, optionally at an elevated temperature; or
c) by hydrolyzing the ester and subsequently treating the corresponding carboxylic acid (R = H) with an appropriate acid, such as, for example, a hydrohalic acid, e.g. hydrochloric acid; sulfuric acid, phosphoric acid; or with a halogenating reagent such as, for example, thionyl chloride.

Said intermediates in turn, can be prepared from an appropriately protected amino acid of formula $R^{1H}$-NH-CH($CH_3$)-COOR (XLVII) wherein R is $C_{1-6}$alkyl or aryl (which is defined an phenyl optionally substituted with from 1-3 substituents independently selected from $C_{1-6}$ alkyl, halo, OH, $C_{1-6}$ alkoxy, $NH_2$, $NO_2$, $CF_3$), by a N-acylation reaction (reaction step L) with an appropriately substituted isatoic anhydride derivative or an appropriate 2-aminobenzoic acid derivative, by stirring the reactants at reflux temperature in an reaction-inert solvent such as, for example, trichloromethane, pyridine.
The intermediates of formula (II-H) may alternatively be prepared from benzodiazepin-2-one derivatives following the procedures described in scheme 4.

Scheme 4

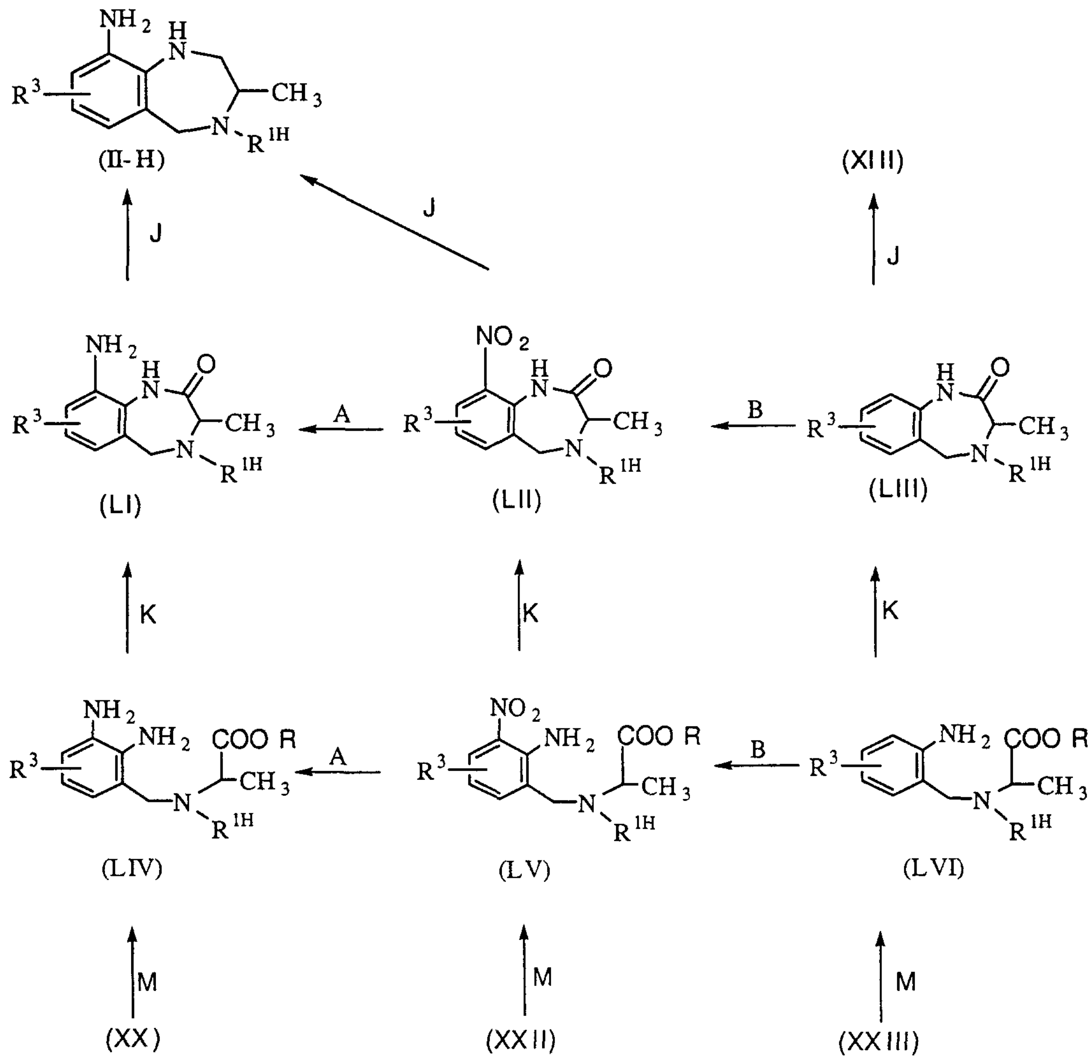


(II-H) (XIII) (LI) (LII) (LIII) (LIV) (LV) (LVI) (XX) (XXII) (XXIII)

**M :** N-alkylation of $R^{1H}$NH-CH($CH_3$)-COOR (XLVI)

The intermediates of formula (IV) can generally be prepared from the compounds of formula (I-b-1) by reacting with a halogenating reagent such as, for example, phosphoryl chloride, phosphorous trichloride, phosphorous tribromide, thionyl chloride, oxalyl chloride, optionally at an elevated temperature, in particular the reflux temperature of the reaction mixture, and optionally in the presence of a base such as, for example, sodium carbonate, sodium hydrogen carbonate, potassium carbonate. The reaction can be conducted in an excess of the halogenating reagent as solvent and optionally a reaction-inert solvent such as an aromatic hydrocarbon or an ether may be used as well.

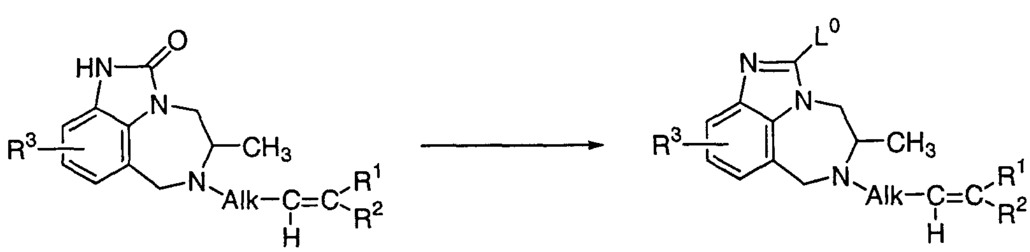

(I-b-1) (IV)

The intermediates of formula (VI) can be prepared from intermediates of formula (II-a) following the condensation reaction with a reagent of formula L-C(=X)-L (II) as described hereinbefore for the preparation of the compounds of formula (I) from the intermediates of formula (II).

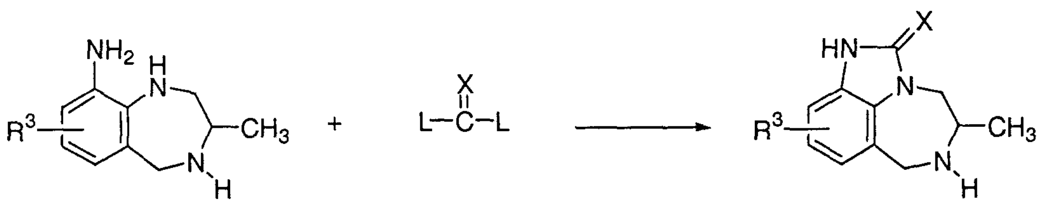

(II-a) (II) (VI)

The intermediates of formula (VI) can be obtained from a benzylated compound of formula (I-c) following art-known hydrogenolysis procedures.

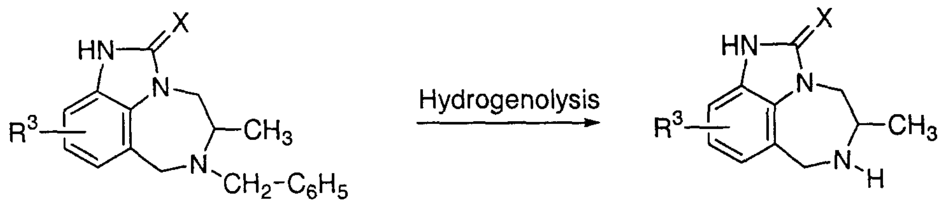


(I-c) (VI)

Said debenzylation reaction can be accomplished by stirring a compound of formula (I-c) in an appropriate reaction-inert solvent in the presence of a suitable metal catalyst and under a hydrogen atmosphere. Appropriate solvents are, for example, alkanols, e.g. methanol, ethanol; carboxylic esters, e.g. ethyl acetate; carboxylic acids, e.g. acetic acid, propanoic acid. As examples of suitable metal catalysts there may be mentioned palladium-on-charcoal, platinum-on-charcoal. In order to prevent the further hydrogenation of the starting material and/or the reaction product it may be appropriate to add a catalyst-poison to the reaction mixture such as, for example thiophene.

The intermediates of formula (VI), wherein X is S, said intermediates being represented by formula (VI-b-2), may be prepared by thionation of an intermediate of formula (VI-b-1) following the procedures described hereinabove for the preparation of the compounds of formula (I-b-2) from (I-b-1).

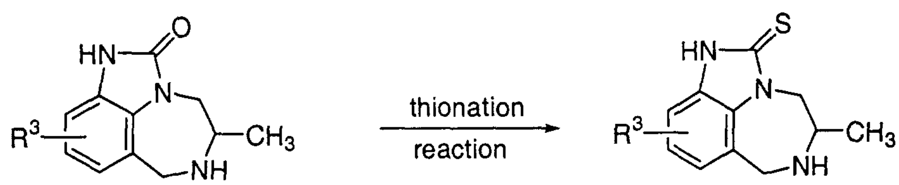


(VI-b-1) (VI-b-2)

The intermediates of formula (XII) wherein $R^{1H}$ is hydrogen, said intermediates being represented by (XII-a) can also be obtained by reacting an appropriately substituted nitrobenzene (LX) and a diamino reagent of formula (LXI). Herein Y is either hydrogen or a removable protective group such as, for example, $C_{1-6}$alkylcarbonyl, e.g. acetyl, trichloroacetyl, a benzyl group, a $C_{1-6}$alkyloxycarbonyl group, e.g. 1,1-dimethylethyloxycarbonyl, groups commonly used to protect an amino group.

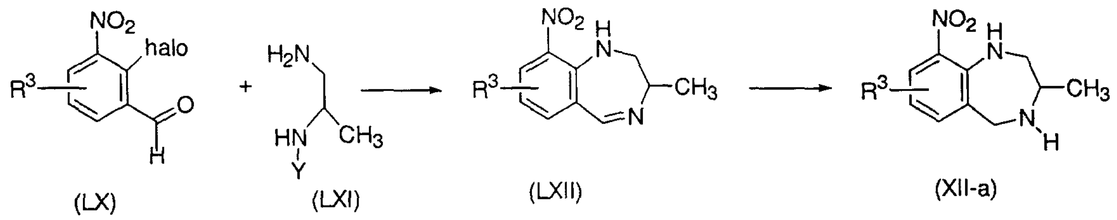

(LX) (LXI) (LXII) (XII-a)

Said reaction may conveniently be conducted by condensing the diamino reagent of formula (LXI) with the nitrobenzene of formula (LX), optionally removing the protective group by alkaline or acid hydrolysis or by catalytic hydrogenation and reducting the thus obtained intermediate (LXII). Said condensation reaction can conveniently be conducted in a suitable reaction-inert solvent such as, for example, an alkanol, e.g. methanol, ethanol, 2-propanol, 1-butanol; an aromatic hydrocarbon, e.g. benzene, methylbenzene, dimethylbenzene; a halogenated hydrocarbon, e. g. trichloromethane, tetrachloromethane; an ether, e.g. tetrahydofuran, 1,4-dioxane, 1,1'-oxybisbutane, 1,1'-oxy (2-methoxyethane); a ketone, e.g. 2-propanone, 4-methyl-2-pentanone; a dipolar aprotic solvent, e.g. N,N-dimethylformamide, N,N-dimethylacetamide, dimethyl sulfoxide; or a mixture of such solvents. It may be appropriate to add a base such as an alkali metal or earth alkaline metal carbonate, e.g. sodium carbonate, sodium hydrogen carbonate, to the reaction mixture. Said condensation reaction can conveniently be conducted at an elevated temperature, in particular at the reflux tempera-ture of the reaction mixture. Said reductions in the above procedure may conveniently be conducted by reacting the intermediate imines with a suitable reductive reagent such as, for example, sodium borohydride, sodium cyanoborohydride.

In all of the foregoing reaction schemes, the chemical designation of the intermediates defines the mixture of all possible stereochemically isomeric forms; mixtures of a number of possible stereochemically isomeric forms such as, for example, diastereomeric mixtures, enantiomeric mixtures, e.g. racemates and enriched enantiomeric mixtures; and the enantiomerically pure isomeric forms of the basic molecular structure.

Stereochemically isomeric forms of the intermediates described in the foregoing reaction schemes and of the compounds of formula (I) may be obtained by the application of art-known procedures. For example, diastereoisomers may be separated by physical separation methods such as destillation, selective crystallization, chromatographic techniques, e.g. counter current distribution, liquid chromatography and the like techniques.

Enantiomerically pure intermediates can conviently be obtained from the enantiomerically pure isomeric forms of the appropriate starting materials, provided that the subsequent reactions occur stereospecifically. Particularly interesting enantiomerically pure starting materials for use in the foregoing reaction schemes are aminoacids and/or substituted derivatives thereof, having the formula $R^{1H}NH-CH(CH_3)-COOR$ (XLVII), and the corresponding aminoalkanols and/or substituted derivatives thereof, having the formula $R^{1H}NH-CH(CH_3)-CH_2OH$ (XXI).

Alternatively, enantiomerically pure intermediates may also be obtained by separating the corresponding racemates for example, by the selective crystallization of their diastereomeric salts with optically active resolving agents, chromatography of diastereomeric derivates, chromatography of the racemate over a chiral stationary phase and the like techniques.

The compounds of formula (I) show antiviral and in particular antiretroviral properties. Until recently, retroviruses were considered to be the pathogenic agents in a number of non-human warm-blooded animal diseases only, unlike viruses which have been known for quite some time to be the cause of a large number of diseases in warm-blooded animals and humans alike. However, since it has been established that a retrovirus, Human Immunodeficiency Virus (HIV), also known as LAV, HTLV-III or ARV, is the etiological agent of Acquired Immune Deficiency Syndrome (AIDS) in humans, retroviral infections and the treatment of subjects suffering therefrom have received the utmost attention. The HIV virus preferentially infects human T-4 cells and destroys them or changes their normal function, particularly the coordination of the immune system. As a result, an infected patient has an everdecreasing number of T-4 cells, which moreover behave abnormally. Hence, the immunological defense system is unable to combat infections and neoplasms and the HIV infected subject usually dies by opportunistic infections such as pneumonia, or by cancers, rather than as a direct result of HIV infections. Other conditions associated with HIV infection include thrombocytopaenia, Kaposi's sarcoma and infection of the central nervous system characterized by progressive demyelination, resulting in dementia and symptoms such as, progressive dysarthria, ataxia and disorientation. HIV infection further has also been associated with peripheral neuropathy, progressive generalized lymphadenopathy (PGL) and AIDS-related complex (ARC). The antiviral, in particular antiretroviral and especially the anti-HIV properties of the compounds of formula (I) suggest said compounds to be useful antiviral chemotherapeutical agents for the prophylaxis or treatment of warm-blooded animals suffering from viral infections, more particularly for the treatment of humans infected by HIV virus.

Due to their antiviral and in particular their antiretroviral properties, the compounds of formula (I), their pharmaceutically acceptable salts and the stereochemically isomeric forms thereof, are useful in the treatment of warm-blooded animals infected with viruses, in particular retroviruses or for the prophylaxis of said warm-blooded animals. In general, the compounds of the present invention may be useful in the treatment of warm-blooded animals infected with viruses whose existence is mediated by, or depends upon, the enzyme reverse transcriptase. Examples of human retroviral infections include HIV and HTLV-I (human T-lymphotropic virus type I), causing leukemia and lymphoma. As an example of non-human animal retroviral infection there may be mentioned FeLV (feline leukemia virus) which causes leukemia and immunodeficiency. Conditions which may be prevented or treated with the compounds of the present invention, especially conditions associated with HIV and other pathogenic retroviruses, include AIDS, AIDS-related complex (ARC), progressive generalized lymphadenopathy (PGL), as well as chronic CNS diseases caused by retroviruses, such as, for example HIV mediated dementia and multiple sclerosis.

In view of their antiviral, in particular antiretroviral activity, the subject compounds may be formulated into various pharmaceutical forms for administration purposes. To prepare the pharmaceutical compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols in the case of oral liquid preparations such as suspensions, syrups, elixirs and solutions: or solid carriers such as starches, sugars, kaolin, lubricants, binders, disintegrating agents in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions.It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls, and segregated multiples thereof.

The present invention is also related with a method of treating viral diseases in warm-blooded animals suffering from said viral diseases by administering an effective antiviral amount of a compound of formula (I), a pharmaceutically acceptable acid addition salt or a stereoisomeric form thereof. Those of skill in the treatment of viral diseases could easily determine the effective antiviral amount from the test results presented herein. In general it is contemplated that an effective amount would be from 0.1 mg/kg to 200 mg/kg body weight, and in particular from 1 mg/kg to 50 mg/kg body weight. It may be appropriate to administer the required dose as two, three, four or more sub-doses at appropriate intervals throughout the day. Said sub-doses may be formulated as unit dosage forms, for example, containing 1 to 1000 mg, and in particular 5 to 200 mg of active ingredient per unit dosage form.

The following examples are intended to illustrate the invention in all its aspects. Unless otherwise stated, all parts therein are by weight.

## Experimental part

### A. Preparation of the intermediates

#### Example 1

a) A solution of 2.6 parts of methyl 2-bromo-3-nitrobenzoate, 1.75 parts of N-[(2-amino-1-methyl)ethyl]benzenemethanamine and 1.06 parts of sodium carbonate in 8 parts of 1-butanol was stirred for 1/2 hour at reflux temperature. The reaction mixture was evaporated and the residue was diluted with 20 parts of water. The product was extracted with trichloromethane (2x30 parts) and the combined extracts were dried, filtered and evaporated. The residue was converted into the hydrochloride salt. The product was filtered off, washed with 2-propanol and dried, yielding 3.4 parts (89.5%) of methyl 2-[[2-methyl-2-[(phenylmethyl)amino]ethyl]amino]-3-nitrobenzoate hydrochloride; mp. 204°C (interm. 1).

b) A mixture of 3.8 parts of intermediate 1; 15 parts of a sodium hydroxide solution 2 N and 4 parts of 2-propanol was stirred for 1 hour at reflux temperature. While refluxing, there was added a solution of 3 parts of concentrated hydrochloric acid and 5 parts of water. After cooling, the precipitated product was filtered off, washed with water and recrystallized from acetic acid, yielding 3 parts (82%) of 2-[[[2-methyl-2-[(phenyl-methyl)amino]ethyl]amino]-3-nitrobenzoic acid; mp. 227°C (interm. 2).

c) A mixture of 189.3 parts of intermediate 2; 400 parts of thionyl chloride and 400 parts of methylbenzene was stirred for 2 hours at reflux temperature. The reaction mixture was evaporated and the residue was taken up in 600 parts of methylbenzene. The whole was neutralized with $NaHCO_3$ (aq.). The organic layer was separated, dried, filtered and concentrated. The residue was left at room temperature. The precipitate was filtered off, washed with 2-propanol and 1,1'-oxybisethane and dried, yielding 123.5 parts of product. The mother liquor was evaporated and the residue was recrystallized from boiling 2-propanol. The product was filtered off at room temperature, washed with 2-propanol and 1,1'-oxybisethane and dried, yielding an additional 28 parts of product. The combined crops were recrystallized from ethanol, yielding 137 parts (85%) of 2,3,4,5-tetrahydro-3-methyl-9-nitro-4-(phenylmethyl)-1H-1,4-benzodiazepin-5-one; mp. 125°C (interm. 3).

d) To a stirred and refluxing suspension of 14 parts of lithium aluminum hydride in 40 parts of benzene and 50 parts of tetrahydrofuran there was added a solution of 20.2 parts of intermediate 3 in 200 parts of tetrahydrofuran. Stirring at reflux temperature was continued for 2.5 hours. After cooling on ice, there were added successively water, NaOH 15% and water. The whole was filtered and the filtrate was evaporated. The residue was co-evaporated with 40 parts of methylbenzene, yielding 19.8 parts (87.6%) of 9-amino-2,3,4,5-tetrahydro-3-methyl-4-(phenylmethyl)-1H-1,4-benzodiazepine (which was used without further purification in the next reaction step) (interm. 4).

e) A mixture of 19.8 parts of intermediate 4 and 7.2 parts of urea was heated at 210-220°C until foaming and evolution of gaseous ammonia ceased (about 10 min). After cooling to 100°C, there were added 120 parts of HCl 1 N. The solution was decanted from the oily residue, boiled with activated charcoal and filtered. After cooling, the filtrate was basified with ammonium hydroxide and extracted with trichloromethane (75 and 150 parts). The combined extracts were dried, filtered and evaporated. The residue was triturated in 2-propanol and was then crystallized from ethanol and from 4-methyl-2-pentanone. The product was filtered off and dried, yielding 2.5 parts (11.5%) of 4,5,6,7-tetrahydro-5-methyl-6-(phenylmethyl)-imidazo[4,5,1-jk][1,4]-benzodiazepin-2(1H)-one; mp. 205°C (interm. 5).

f) A mixture of 8 parts of intermediate 5 in 80 parts of acetic acid was hydrogenated at normal pressure and 38°C with 1 part of palladium-on-charcoal catalyst 10%. After the calculated amount of hydrogen was taken up, the catalyst was filtered off and the filtrate was evaporated. The residue was taken up in 75 parts of water and the whole was basified with 30 parts of concentrated ammonium hydroxide. The mixture was left to crystallize at room temperature. The product was filtered off and recrystallized from 2-propanol, yielding 3.7 parts (66.8%) of 4,5,6,7-tetrahydro-5-methylimidazo[4,5,1-jk] [1,4]benzodiazepin-2(1H)-one; mp. 190.5°C (interm. 6).

Example 2

a) To a stirred and cooled (-12°C) mixture of 9.10 parts of 2-amino-3-nitrobenzoic acid, 6.95 parts of methyl L-α-alanine monohydrochloride, 13.50 parts of 1-hydroxy-1H-1,2,4-benzotriazole monohydrate and 178 parts of tetrahydrofuran there were added portionwise 5.05 parts of N-methylmorpholine and, after 5 min, 10.3 parts of N,N'-methanetetraylbis[cyclohexanamine] under an argon atmosphere. Stirring was continued for 5 1/2 hours at -12°C and for 15 hours at room temperature. After cooling to 0°C for 1/2 hour, the reaction mixture was filtered and the filtrate was evaporated. The residue was partitioned between ethyl acetate and a saturated sodium hydrogen carbonate solution. The organic layer was separated, washed with $NaHCO_3$ (sat.), dried, filtered and evaporated. The residue was triturated with hexane. The product was filtered off and dried, yielding 13.08 parts (97.9%) of (-)-methyl (S)-2-[(2-amino-3-nitrobenzoyl)-amino]propanoate; mp. 132.9°C (interm. 7).

b) A mixture of 12.58 parts of intermediate 7; 3.50 parts of palladium-on-charcoal catalyst 10% and 158 parts of ethanol was hydrogenated in a Parr apparatus for 4 hours at room temperature and a pressure of $3.1\ 10^5$ Pa. The catalyst was filtered off over diatomaceous earth and the filtrate was evaporated. The residue was placed under reduced pressure ($3.3\ 10^3$ Pa.) and stirred at 150°C for 10 min and at 202°C for 40 min. After cooling, the solid

was triturated with ethanol. The product was filtered off, washed with ethanol and 1,1'-oxybisethane and dried, yielding 5.58 parts (57.7%) of (+)-(S)-9-amino-3,4-dihydro-3-methyl-1H-1,4-benzodiazepine-2,5-dione (interm. 8).

c) To a suspension of 5.55 parts of lithium aluminum hydride in 154.5 parts of 1,4-dioxane there were added 5.00 parts of intermediate 8 under an argon atmosphere. After refluxing for 5 hours and subsequent cooling to 10°C, there were added successively 5.55 parts of water, 9.16 parts of NaOH 15% and 16.65 parts of water. The whole was stirred for 2 hours and then filtered. The precipitate was washed with 178 parts of hot tetrahydrofuran and 133 parts of hot dichloromethane. The combined filtrates were dried, filtered and evaporated. The residue was poured into a solution of 7.36 parts of N-methylmorpholine in 133 parts of dichloromethane. The whole was added dropwise to a solution of 4.82 parts of trichloromethyl chloroformate in 160 parts of dichloromethane at 0°C under an argon stream. Stirring at 0°C was continued for 10 min. After warming to room temperature, the reaction mixture was evaporated and there were added 70 parts of an aqueous 1,4-dioxane solution (15%) to the residue. The mixture was heated on a steam-bath for 45 min under a nitrogen stream, cooled and extracted with dichloromethane (2x66.5 parts). The aqueous layer was filtered and basified with concentrated ammonium hydroxide. The precipitate was filtered off, washed with cold water, dried and triturated with 2-propanol (2x), yielding 1.59 parts (32.1%) of (+)-(S)-4,5,6,7-tetrahydro-5-methylimidazo[4,5,1-jk][1,4]benzodiazepin-2(1H)-one; mp. 206.5°C (interm. 9).

In a similar manner there was also prepared (+)-(S)-4,5,6,7-tetrahydro-5,8-dimethylimidazo[4,5,1-jk][1,4]benzodiazepin-2(1H)-one; mp. 207.8°C (interm. 10).

d) A mixture of 1.50 parts of intermediate 9; 1.11 parts of bromomethylcyclopropane, 1.18 parts of sodium carbonate, 1.22 parts of potassium iodide and 28.2 parts of N,N-dimethylformamide was heated at 85°C for 24 hours under an argon atmosphere. The reaction mixture was evaporated and the residue was partitioned between water and dichloromethane. The organic layer was separated, washed with NaOH 3N and NaCl (sat.), dried, filtered and evaporated. To the residual oil there were added 3.95 parts of acetonitrile. After cooling at 0°C for 1 hour, the product was filtered off, washed with acetonitrile and further purified by flash column chromatography (silica gel ; $CH_2Cl_2$ / $C_2H_5OH$ 97:3). The eluent of the desired fraction was evaporated, yielding 0.77 parts (40.5%) of (+)-(S)-6-(cyclopropylmethyl)-4,5,6,7-tetrahydro-5-methylimidazo-[4,5,1-jk] [1,4]benzodiazepin-2(1H)-one; mp. 115.9°C (interm. 11).

e) To a stirred and cooled (-60 to -50°C) amount of 45.3 parts of concentrated nitric acid there were added portionwise 2.75 parts of intermediate 11 under an argon atmosphere. When a clear solution was obtained, stirring and cooling was continued for 1/2 hour. The reaction mixture was slowly poured into 400 parts of ice-water and the whole was basified to pH 8 with $Na_2CO_3$. The precipitated product was filtered off and dried in vacuo at 50°C for 16 hours, yielding 0.5 parts (15.6%) of a mixture of (S)-6-(cyclo-propylmethyl)-4,5,6,7-tetrahydro-5-methyl-9-nitroimidazo[4,5,1-jk][1,4]benzodiazepin-2(1H)-one and the 8-nitro-isomer thereof (75:25) (interm. 12).

f) To a refluxing mixture of 1.03 parts of hydrazine monohydrate, 23.7 parts of methanol and 0.15 parts of Raney nickel there were added portionwise 0.5 parts of intermediate 12. After refluxing for 20 min and subsequent cooling, the reaction mixture was filtered over diatomaceous earth and the filtrate was evaporated. The residue was purified by preparative thin layer chromatography (eluens : $CH_2Cl_2$ / $CH_3OH$ 90:10). The eluent of the pure fractions was evaporated and the residue was dried in vacuo at 50°C for 16 hours, yielding 0.17 parts (39.0%) of (+)-(S)-9-amino-6-(cyclopropyl-methyl)-4,5,6,7-tetrahydro-5-methylimidazo[4,5,1-jk][1,4]benzodiazepin-2(1H)-one; mp. 188.7°C; $[\alpha]_D^{25}$ = +13.4° (conc. = 0.50% in trichloromethane) (interm. 13).

g) To a refluxing mixture of 1.6 parts of Raney nickel, 15.48 parts of hydrazine monohydrate and 158 parts of methanol there were added portionwise 12.4 parts of intermediate 12. After refluxing for 20 min and subsequent cooling, the reaction mixture was filtered over diatomaceous earth and the filtrate was evaporated. The residue was triturated in acetonitrile and then taken up in trichloromethane. This solution was washed with water, dried, filtered and evaporated. The residue was crystallized from acetonitrile. After filtration, the mother liquor was evaporated and the residue was purified by column chromatography (silica gel ; $CH_2Cl_2$ /$CH_3OH$(10% $NH_4OH$) 99:1 Ø 97:3 Ø 95:5). The eluent of the desired fractions was evaporated and the residue was dried for 16 hours at 50 °C, yielding 1.25 parts (11.2%) of (+)-(S)-8-amino-6-(cyclopropylmethyl)-4,5,6,7-tetrahydro-5-methylimidazo[4,5,1-jk] [1,4]benzodiazepin-2(1H)-one;
mp.206.0 °C; $[\alpha]_D^{25}$ = +4.5° (c = 0.44% in methanol) (interm. 14).

Example 3

a) A mixture of 41.49 parts of 6-chloro-1H-3,1-benzoxazine-2,4-dione and 31.40 parts of methyl L-α-alanine monohydrochloride in 108 parts of pyridine was refluxed for 10 hours under an argon atmosphere. After cooling, the reaction mixture was stirred for 12 hours at room temperature. The precipitate was filtered off, rinsed with water and triturated in ethanol. The product was filtered off, washed with ethanol and dried, yielding 24.77 parts (52.5%)

of (S)-7-chloro-3,4-dihydro-3-methyl-1H-1,4-benzodiazepine-2,5-dione (interm. 15).

b) To a cooled (0°C) amount of 142 parts of nitric acid there were added portionwise 24.55 parts of intermediate 15 under an argon atmosphere. After cooling for 3 1/2 hours at 0°C, the reaction mixture was slowly added to 450 parts of ice while stirring. The precipitate was filtered off, rinsed with water and dried at room temperature overnight, yielding 27.84 parts (93.9%) of (S)-7-chloro-3,4-dihydro-3-methyl-9-nitro-1H-1,4-benzodiazepine-2,5-dione (interm. 16).

c) To a cooled (0°C) suspension of 18.2 parts of lithium aluminum hydride in 261 parts of 1,2-dimethoxyethane there were added portionwise 16.14 parts of intermediate 16 under a nitrogen atmosphere. The mixture was stirred for 2 hours at 0°C and for 40 hours at reflux temperature. After cooling to 0°C, there were added a mixture of 18.2 parts of water and 48.1 parts of tetrahydrofuran, 21.1 parts of NaOH 15% and 54.6 parts of water. The mixture was stirred for 1 hour at room temperature and was then filtered. The precipitate was refluxed in tetrahydrofuran for 5 min and filtered off again. The combined filtrates were dried, filtered and evaporated and the residue was dissolved in 399 parts of dichloromethane. After drying and filtering, this solution was combined with 18.2 parts of N-methylmorpholine and the whole was added dropwise to a mixture of 11.9 parts of trichloromethyl chloroformate and 665 parts of dichloromethane at 0°C and under argon. The whole was evaporated and the residue was taken up in 150 ml of a mixture of water and 1,4-dioxane 85:15. The mixture was heated for 2 hours on a steam-bath under nitrogen. After cooling, the solid was filtered off and dissolved in 80 parts of water. The solution was basified with $NH_4OH$ and stirred for 45 min. The product was filtered off and crystallized successively from acetonitrile and 2-propanol, yielding 2.28 parts (16%) of (+)-(S)-9-chloro-4,5,6,7-tetrahydro-5-methylimidazo[4,5,1-jk] [1,4]benzodiazepin-2(1H)-one; mp. 202.2°C; $[\alpha]_D^{20}$ = +72.6° (conc. = 0.98% in methanol) (interm. 17).

In a similar manner there was also prepared 4,5,6,7-tetrahydro-5,9-dimethylimidazo-[4,5,1-jk] [1,4]benzodiazepin-2(1H)-one; mp. 199.2°C (interm. 18).

Example 4

a) To a homogeneous solution of 8.42 parts of (S)-2-aminopropanamide monohydrobromide, 12.26 parts of sodium acetate and 79 parts of methanol there were added 10.96 parts of 2,6-dichloro-3-nitrobenzaldehyde and, after 1/2 hour, a mixture of 3.77 parts of sodium cyanotrihydroborate and 7.9 parts of methanol. The whole was stirred for 45 min at room temperature. After acidifying to pH 1 with HCl 3N, stirring was continued overnight. The reaction mixture was evaporated and the residue was basified with $NaHCO_3$ (sat.). The product was extracted with dichloromethane and the extract was dried, filtered and evaporated. The residue was recrystallized from 2-propanol, yielding 10.29 parts (70.7%) of (S)-2-[[(2,6-dichloro-3-nitrophenyl)methyl]amino]propanamide (interm.19).

b) A mixture of 10.03 parts of intermediate (19), 348 parts of 1,2-dimethoxyethane and 92.5 parts of a solution of borane tetrahydrofurancomplex in tetrahydrofuran 1M was stirred for 3 days at room temperature under argon. There were slowly added 142 parts of methanol and 180 ml of HCl 3N and stirring was continued over weekend. The reaction mixture was basified with 200 ml of NaOH 3N and was then evaporated. The residue was extracted with dichloromethane and the extract was dried, filtered and evaporated. The residue was refluxed in a mixture of 3.0 parts of sodium acetate and 81 parts of 1-butanol for 3 days under argon. The solvent was evaporated and the residue was dissolved in dichloromethane. This solution was washed with $NaHCO_3$, dried, filtered and evaporated. The residue was purified twice by flash column chromatography (silica gel ; $CH_2Cl_2$ / $CH_3OH$ 99:1). The eluent of the desired fraction was evaporated and the residue was converted into the (E)-2-butenedioate salt in methanol. The salt was filtered off (1st fraction) and the mother liquor was evaporated. The residue was chromatographed and also converted into the salt (2nd fraction). The combined fractions were treated with a mixture of dichloromethane and NaOH 3N to set free the base, yielding 3.73 (45.0%) of (S)-6-chloro-2,3,4,5-tetrahydro-3-methyl-9-nitro-1H-1,4-benzodiazepine (interm. 20).

c) To 1.89 parts of intermediate 20 under argon there were added 1.24 parts of sodium carbonate, 1.30 parts of potassium iodide and a solution of 1.46 parts of 1-bromo-3-ethyl-2-pentene in 17.86 parts of N,N-dimethylformamide. After stirring overnight, the residue was taken up in 1,1'-oxybisethane. The whole was washed with water and NaCl (sat.), dried, filtered and evaporated, yielding 2.67 parts (100%) of (S)-6-chloro-4-(3-ethyl-2-pentenyl)-2,3,4,5-tetrahydro-3-methyl-9-nitro-1H-1,4-benzodiazepine (interm.21).

In a similar manner there was also prepared (S)-6-chloro-4-(2-cyclopentylideneethyl)-2,3,4,5-tetrahydro-3-methyl-9-nitro-1H-1,4-benzodiazepine (interm.22).

d) To a cooled(0°C) mixture of 1.16 parts of lithium aluminum hydride and 26.7 parts of tetrahydrofuran under argon there was added dropwise a solution of 2.57 parts of intermediate 21 in 40.05 parts of tetrahydrofuran. The whole was stirred for 1/2 hour at 0 °C, for 1 hour at room temperature and for 8 hours at reflux temperature. Then there were slowly added 1.16 parts of water, 1.16 ml of NaOH 3N, 3.48 parts of water and 35.6 parts of tetrahydrofuran. After stirring for 1/2 hour, the precipitate was filtered off and washed with hot tetrahydrofuran. The combined filtrates were evaporated and the residue was dissolved in dichloromethane. This solution was dried, filtered and evaporated, yielding 2.41 parts (100%) of (S)-6-chloro-4-(3-ethyl-2-pentenyl)-2,3,4,5-tetrahydro-3-methyl-1H-1,4-benzodiazepin-9-amine (interm. 23).

e) To a refluxing mixture of 0.31 parts of Raney nickel, 2.3 parts of intermediate 22 and 71.1 parts of methanol under argon, there were added dropwise 2.12 parts of hydrazine monohydrate. After refluxing for 1/2 hour, the reaction mixture was evaporated and the residue was dissolved in 1,1'-oxybisethane. This solution was washed with water and NaCl (sat.), dried, filtered and evaporated, yielding 2.04 parts (97.2%) of (S)-6-chloro-4-(2-cyclopentylideneethyl)-2,3,4,5-tetrahydro-3-methyl-1H-1,4-benzodiazepin-9-amine (interm.24).

Example 5

A mixture of 4.0 parts of compound 2; 46.2 parts of phosphoryl chloride and 1.30 parts of sodium carbonate was heated at 85°C for 18 hours while nitrogen was bubbled through. The excess of phosphoryl chloride was removed and to the residue there were added successively 100 parts of water, 100 ml of $NaHCO_3$ (sat.) and 266 parts of dichloromethane. The aqueous layer was separated and re-extracted with dichloromethane. The combined organic layers were dried, filtered and evaporated, yielding 1.90 parts (44.9%) of (S)-2,9-dichloro-6-(3-ethyl-2-pentenyl)-4,5,6,7-tetrahydro-5-methyl-imidazo[4,5,1-jk] [1,4]benzodiazepine (interm. 25).

In a similar manner compound 20 was converted into (+)-(S)-2,9-dichloro-6-(2-cyclopentylideneethyl)-4,5,6,7-tetrahydro-5-methyl-imidazo[4,5,1-jk][1,4]-benzodiazepine (interm.26).

Example 6

To a cooled (-78°C) solution of 1.23 parts of compound 18 in 93.1 parts of dichloromethane under argon, there were added successively 1.38 parts of trifluoroacetic anhydride, after 10 min, 0.79 parts of 2,6-dimethylpyridine and, after 15 min, 23.1 ml of a solution of HCl in 1,1'-oxybisethane 0.8N. The whole was left for 15 min and was then poured into $NaHCO_3$ (sat.). The organic layer was separated, dried, filtered and evaporated, yielding 1.61 parts (100%) of (S)-2,8-dichloro-6-(3-ethyl-2-pentenyl)-4,5,6,7-tetrahydro-5-methylimidazo[4,5,1-jk] [1,4]benzodiazepine (interm. 27).

B. Preparation of the final compounds

Example 7 (preparative example)

To a mixture of 1.00 part of intermediate 6; 0.782 parts of sodium carbonate, 0.816 parts of potassium iodide and 94 parts of N,N-dimethylformamide there were added 1.18 parts of 2,3-dibromopropene under an argon atmosphere. The whole was heated at 65-70°C for 5 hours under argon and was then evaporated. The residue was partitioned between dichloromethane and water. The organic layer was separated and washed with water. The combined aqueous layers were re-extracted with dichloromethane. The combined organic layers were washed with NaCl (sat.), dried, filtered and evaporated. The residue was dissolved in refluxing acetonitrile and recrystallized upon cooling (2x). The product was filtered off, washed with cold acetonitrile and dried in vacuo at 82°C, yielding 0.762 parts (48.1%) of 6-(2-bromo-2-propenyl)-4,5,6,7-tetrahydro-5-methylimidazo[4,5,1-jk][1,4]benzodiazepin-2(1H)-one; mp. 150.0°C (comp. 1; compound not claimed).

Example 8

To a mixture of 1.50 parts of intermediate 10; 0.77 parts of sodium carbonate and 9.4 parts of N,N-dimethylformamide there were added 1.47 parts of 1-bromo-3-ethyl-2-pentene. After heating at 60°C for 1 1/2 hour and subsequent cooling, the reaction mixture was partitioned between water and dichloromethane. The organic layer was separated, dried, filtered and evaporated. The residue was crystallized from acetonitrile. The product was filtered off, washed with cold acetonitrile and dried, yielding 0.93 parts (43.0%) of (+)-(S)-6-(3-ethyl-2-pentenyl)-4,5,6,7-tetrahydro-5,8-dimethylimidazo-[4,5,1-jk][1,4]benzodiazepin-2(1H)-one; mp. 117.3°C; $[\alpha]_D^{25}$ = +4.9° (conc. = 1.0% in methanol) (comp. 5).

Example 9

A mixture of 1.90 parts of intermediate 25; 1.50 parts of thiourea and 39.5 parts of ethanol was refluxed for 16 hours. The reaction mixture was evaporated and the residue was taken up in a mixture of 180 parts of ethyl acetate,

50 parts of water and 50 ml of $NaHCO_3$ (sat.). The organic layer was separated, washed with water, dried, filtered and evaporated. The residue was purified by flash column chromatography (silica gel; hexane / $CH_3COOC_2H_5$ 8:1 Ø 4:1). The eluent of the desired fraction was evaporated and the residue was converted into the hydrochloride salt in ethanol, yielding 1.74 parts (83.4%) of (-)-(S)-9-chloro-6-(3-ethyl-2-pentenyl)-4,5,6,7-tetrahydro-5-methylimidazo[4,5,1-jk][1,4] benzodiazepine-2(1H)-thione monohydrochloride; mp. >280°C (decomp.); $[\alpha]_D^{25}$ = -32.7° (conc. = 1.0% in methanol) (comp. 7)

Example 10

To a cooled (0°C) mixture of 0.75 parts of trichloromethyl chloroformate and 33.2 parts of dichloromethane under argon, there was added slowly a solution of 2.39 parts of intermediate 23 in 33.2 parts of dichloromethane. After stirring for 1/2 hour at 0°C, there was added $NaHCO_3$ (sat.). The organic layer was separated, dried, filtered and evaporated. The residue was purified by flash column chromatography (silica gel; $CH_2Cl_2$ / $CH_3OH$ 98.5:1.5). The eluent of the desired fraction was evaporated and the residue was crystallized from acetonitrile. The product was filtered off and dried, yielding 1.12 parts (44.1%) of (+)-(S)-8-chloro-6-(3-ethyl-2-pentenyl)-4,5,6,7-tetrahydro-5-methylimidazo[4,5,1-jk][1,4]benzodiazepin-2(1H)-one; mp. 156.8°C; $[\alpha]_D^{20}$ = +7.15° (c = 0.1% in methanol) (comp. 18).

Example 11

1.60 Parts of intermediate 27, 31.6 parts of ethanol and 3.37 parts of thiourea were combined under argon and refluxed overnight. The solvent was evaporated and the residue was dissolved in 1,1'-oxybisethane. This solution was washed with water, dried, filtered and evaporated. The residue was purified twice by flash column chromatography (silica gel ; $CH_2Cl_2$ / $CH_3OH$ 99:1). The eluent of the desired fraction was evaporated and the residue was crystallized from acetonitrile. The product was filtered off and dried, yielding 0.55 parts (42.6%) of (+)-(S)-8-chloro-6-(3-ethyl-2-pentenyl)-4,5,6,7-tetrahydro-5-methylimidazo[4,5,1-jk] [1,4]benzodiazepine-2(1H)-thione; mp. 113.5°C; $[\alpha]_D^{20}$ = +0.907° (c = 0.1% in methanol) (comp. 19).

All compounds listed in Table 1 were prepared following methods of preparation described in Examples 7-11, as is indicated in the column Ex. No.

Table 1

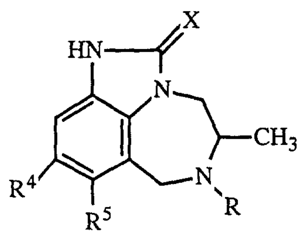

| Co. No. | Ex. No. | X | R | $R^4$ | $R^5$ | physical data |
|---|---|---|---|---|---|---|
| 2 | 8 | O | $-CH_2-CH=C(C_2H_5)_2$ | Cl | H | mp. 239.4°C/(-)(S)/HCl $[\alpha]_D^{25}$ 1% $CH_3OH$ = -7.9° |
| 5 | 8 | O | $-CH_2-CH=C(C_2H_5)_2$ | H | $CH_3$ | mp. 117.3°C/(+)(S) $[\alpha]_D^{25}$ 1% $CH_3OH$ = +4.9° |
| 6 | 7 | O | $-CH_2-CH=C(C_2H_5)_2$ | $CH_3$ | H | mp. 103.0°C/(±) |
| 7 | 9 | S | $-CH_2-CH=C(C_2H_5)_2$ | Cl | H | mp. >280°C/(-)(S)/HCl $[\alpha]_D^{25}$ 1% $CH_3OH$ = -32.7° |
| 10 | 8 | O* | $-CH_2-CH=C(C_3H_7)_2$ | Cl | H | mp. 200.5°C/(-)(S)/HCl $[\alpha]_D^{25}$ 1% $CH_3OH$ = -8.2° |
| 15 | 8 | O | $-CH_2-CH=C[CH(CH_3)_2]_2$ | Cl | H | mp. 205.8°C/(-)-(S)/HCl $[\alpha]_D^{25}$ 1% $CH_3OH$ = -4.0° |
| 18 | 10 | O | $-CH_2-CH=C(C_2H_5)_2$ | H | Cl | mp. 156.8°C/(+)-(S) $[\alpha]_D^{20}$ 0.1% $CH_3OH$ = +7.15° |
| 19 | 11 | S | $-CH_2-CH=C(C_2H_5)_2$ | H | Cl | mp. 113.5°C/(+)-(S) $[\alpha]_D^{20}$ 0.1% $CH_3OH$ = +0.91° |

* : $CH_2Cl_2$ / $CH_3OH$ / $NH_4OH$ (100:5:0.5)

C. Pharmacological example

Example 12

A rapid, sensitive and automated assay procedure was used for the in-vitro evaluation of anti-HIV agents. An HIV-1 transformed T4-cell line, MT-4, which was previously shown (Koyanagi et al., Int. J. Cancer, 36, 445-451, 1985) to be highly susceptible to and permissive for HIV infection, served as the target cell line. Inhibition of the HIV-induced cytopathic effect was used as the end point. The viability of both HIV- and mock-infected cells was assessed spectrophotometrically via the in-situ reduction of 3-(4,5-dimethylthiazol-2-yl)-2,5-diphenyltetrazolium bromide (MTT). The 50% cytotoxic dose ($CD_{50}$ in µg/ml) was defined as the concentration of compound that reduced the absorbance of the mock-infected control sample by 50%. The percent protection achieved by the compound in HIV-infected cells was calculated by the following formula:

$$\frac{(OD_T)_{HIV} - (OD_C)_{HIV}}{(OD_C)_{MOCK} - (OD_C)_{HIV}}$$

expressed in %,
whereby $(OD_T)_{HIV}$ is the optical density measured with a given concentration of the test compound in HIV-infected cells; $(OD_C)_{HIV}$ is the optical density measured for the control untreated HIV-infected cells; $(OD_C)_{MOCK}$ is the optical density measured for the control untreated mock-infected cells; all optical density values were determined at 540 nm. The dose achieving 50% protection according to the above formula was defined as the 50% effective dose ($ED_{50}$ in µg/ml). The ratio of $CD_{50}$ to $ED_{50}$ was defined as the selectivity index (SI).

Table 2 :

| 50% cytotoxic ($CD_{50}$), 50% effective dose ($ED_{50}$) and selectivity index (SI). | | | |
|---|---|---|---|
| Comp. No. | $CD_{50}$ (µg/ml) | $ED_{50}$ (µg/ml) | SI |
| 2 | >10 | 0.032 | >312 |
| 5 | 14.3 | 0.038 | 370 |
| 7 | 5.2 | 0.006 | 850 |

D. Composition Examples

Example 13 : ORAL SOLUTION

9 g of methyl 4-hydroxybenzoate and 1 g of propyl 4-hydroxybenzoate are dissolved in 4 1 of boiling purified water. In 3 l of this solution are dissolved first 10 g of 2,3-di-hydroxybutanedioic acid and thereafter 20 g of the A.I. The latter solution is combined with the remaining part of the former solution and 12 l 1,2,3-propanetriol and 3 l of sorbitol 70% solution are added thereto. 40 g of sodium saccharin are dissolved in 0.5 l of water and 2 ml of raspberry and 2 ml of gooseberry essence are added. The latter solution is combined with the former, water is added q.s. to a volume of 20 l providing an oral solution comprising 5 mg of the active ingredient per teaspoonful (5 ml). The resulting solution is filled in suitable containers.

Example 14: CAPSULES

20 g of the A.I., 6 g sodium lauryl sulfate, 56 g starch, 56 g lactose, 0.8 g colloidal silicon dioxide, and 1.2 g magnesium stearate are vigorously stirred together. The resulting mixture is subsequently filled into 1000 suitable hardened gelatin capsules, comprising each 20 mg of the active ingredient.

Example 15 : FILM-COATED TABLETS

Preparation of tablet core

A mixture of 100 g of the A.I., 570 g lactose and 200 g starch is mixed well and thereafter humidified with a solution of 5 g sodium dodecyl sulfate and 10 g polyvinylpyrrolidone (Kollidon-K 90 ®) in about 200 ml of water. The wet powder mixture is sieved, dried and sieved again. Then there are added 100 g microcrystalline cellulose (Avicel ®) and 15 g hydrogenated vegetable oil (Sterotex ®). The whole is mixed well and compressed into tablets, giving 10.000 tablets, each containing 10 mg of the active ingredient.

Coating

To a solution of 10 g methyl cellulose (Methocel 60 HG®) in 75 ml of denaturated ethanol there is added a solution of 5 g of ethyl cellulose (Ethocel 22 cps ®) in 150 ml of dichloromethane. Then there are added 75 ml of dichloromethane and 2.5 ml 1,2,3-propanetriol. 10 g of polyethylene glycol is molten and dissolved in 75 ml of dichloromethane. The latter solution is added to the former and then there are added 2.5 g of magnesium octadecanoate, 5 g of polyvinylpyrrolidone and 30 ml of concentrated colour suspension (Opaspray K-1-2109®) and the whole is homogenated. The tablet cores are coated with the thus obtained mixture in a coating apparatus.

Example 16: INJECTABLE SOLUTION

1.8 g methyl 4-hydroxybenzoate and 0.2 g propyl 4-hydroxybenzoate are dissolved in about 0.5 1 of boiling water for injection. After cooling to about 50°C there are added while stirring 4 g lactic acid, 0.05 g propylene glycol and 4 g of the A.I.. The solution is cooled to room temperature and supplemented with water for injection q.s. ad 1 l, giving a solution comprising 4 mg/ml of A.I.. The solution is sterilized by filtration (U.S.P. XVII p. 811) and filled in sterile containers.

## Claims

**Claims for the following Contracting States : AT, SE**

1. A compound having the formula :

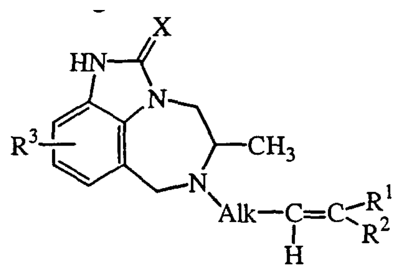

(I),

a pharmaceutically acceptable acid addition salt or a stereochemically isomeric form thereof, wherein

X is O or S;
Alk is $C_{1-6}$alkanediyl;
$R^1$ and $R^2$ are $C_{1-4}$alkyl
$R^3$ is methyl or chloro

2. A compound according to claim 1 wherein X represents O.

3. A compound according to claim 1 wherein X represents S.

4. A compound according to claim 1 wherein the compound is (+)-(S)-8-chloro-6-(3-ethyl-2-pentenyl)-4,5,6,7-tetrahydro-5-methylimidazo[4,5,1-jk][1,4]benzodiazepin-2(1H)-thione.

5. An antiviral composition comprising a pharmaceutically acceptable carrier and as active ingredient an effective antiviral amount of a compound as claimed in any of claims 1-4.

6. A method of preparing a pharmaceutical composition as claimed in claim 5, **characterized in that** a therapeutically effective amount of a compound as claimed in any of claims 1 to 4 is intimately mixed with a pharmaceutical carrier.

7. A compound as claimed in any of claims 1 to 4 for use as a medicine.

8. A process of preparing a compound as claimed in any of claims 1 to 4, **characterized by**

a) condensing a 9-amino-2,3,4,5-tetrahydro-1H-1,4-benzodiazepine of formula :

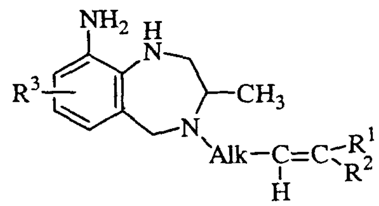

(II),

wherein $R^1$, $R^2$, $R^3$, and Alk are as defined in formula (I) with a reagent of formula L-C(=X)-L (III) wherein L is a reactive leaving group and X is O or S, in a reaction-inert solvent;

b) reacting a 4,5,6,7-tetrahydroimidazo[4,5,1-jk][1,4]benzodiazepine of formula :

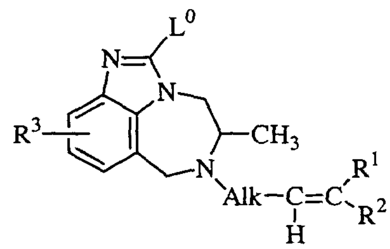

(IV)

wherein $R^1$, $R^2$, $R^3$, and Alk are as defined in formula (I) and $L^0$ is a reactive leaving group with a reagent of formula $M_2$-X (V), wherein X is as defined in formula (I), in a reaction-inert solvent or in the presence of an excess of the reagent of formula (V), optionally in a reaction-inert solvent;

c) N-alkylating an intermediate of formula

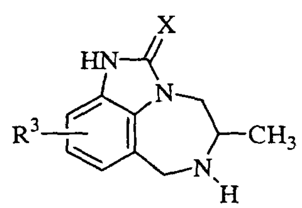

(VI)

wherein $R^3$ and X are as defined in formula (I) with a reagent of formula $R^1R^2C$=CH-Alk-W (VII) wherein W represents a reactive leaving group and $R^1$ and $R^2$ are as defined in formula (I), in a reaction-inert solvent;

d) cyclizing a benzimidazole of formula :

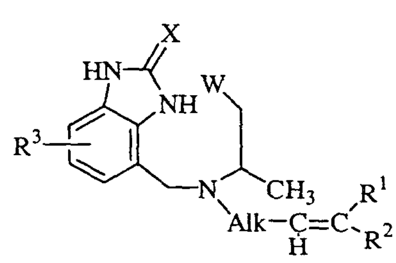

(XI)

wherein $R^1$, $R^2$, $R^3$, Alk and X are as defined in formula (I) and W represents a reactive leaving group, in a reaction-inert solvent, optionally in the presence of a base and optionally at an elevated temperature; and, if desired, converting the compounds of formula (I) into a therapeutically active non-toxic acid addition salt form by treatment with an acid; or conversely, converting the acid salt into the free base with alkali; and/or

preparing stereochemically isomeric forms thereof.

**9.** A process of preparing a compound as claimed in any of claims 1,3 or 4 and wherein X represents S, **characterized by**

a) thionating a compound of formula :

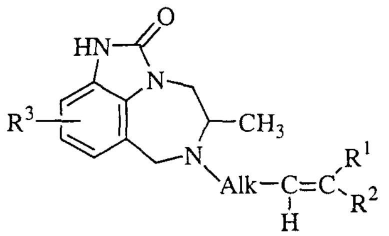
(I-b-1)

wherein $R^1$, $R^2$, $R^3$ and Alk are as defined in formula (I) with phosphorus pentasulfide in a reaction-inert solvent, thus yielding a compound of formula :

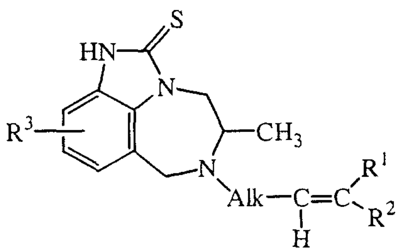
(I-b-2)

b) thiating a tetrahydroimidazo[4,5,1-jk][1,4]benzodiazepine of formula :

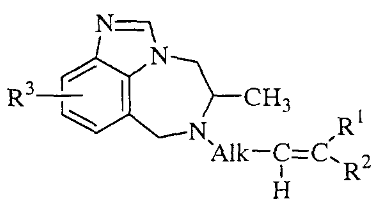
(IX)

wherein $R^1$, $R^2$, $R^3$ and Alk are as defined in formula (I) with elemental sulfur at an elevated temperature, thus yielding a compound of formula (I-b-2);

c)reducing and thiocarbonylating a 9-nitrobenzodiazepine of formula :

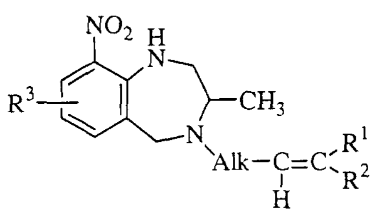
(X)

wherein $R^1$, $R^2$, $R^3$ and Alk are as defined in formula (I) in the presence of an alkali metal sulfide or hydrogen sulfide, and carbon disulfide, thus yielding a compound of formula (I-b-2); and if desired, converting the compounds of formula (I) into a therapeutically active non-toxic acid addition salt form by treatment with an acid; or conversely, converting the acid salt into the free base with alkali; and/or preparing stereochemically isomeric forms thereof.

**Claims for the following Contracting States : ES, GR**

**1.** A process for preparing a compound having the formula:

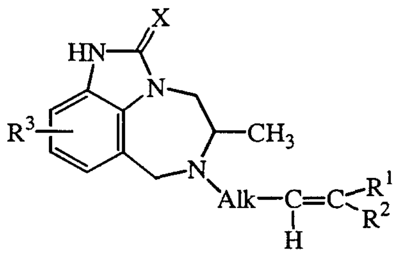

(I),

a pharmaceutically acceptable acid addition salt or a stereochemically isomeric form thereof, wherein

X is O or S;
Alk is $C_{1-6}$alkanediyl;
$R^1$ and $R^2$ are $C_{1-4}$alkyl;
$R^3$ is methyl or chloro;

**characterized by** :

a) condensing a 9-amino-2,3,4,5-tetrahydro-1H-1,4-benzodiazepine of formula :

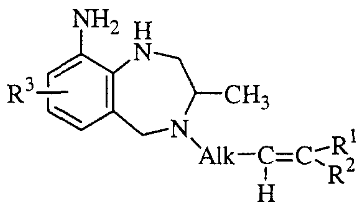

(II),

wherein $R^1$, $R^2$, $R^3$, and Alk are as defined in formula (I) with a reagent of formula L-C(=X)-L (III) wherein L is a reactive leaving group and X is O or S, in a reaction-inert solvent;

b) reacting a 4,5,6,7-tetrahydroimidazo[4,5,1-jk][1,4]benzodiazepine of formula :

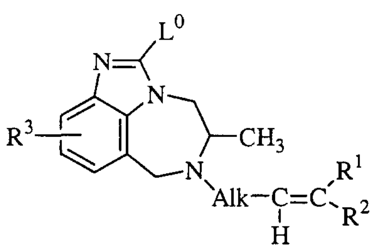

(IV)

wherein $R^1$, $R^2$, $R^3$, and Alk are as defined in formula (I) and $L^0$ is a reactive leaving group with a reagent of formula $M_2$-X (V), wherein X is as defined in formula (I), in a reaction-inert solvent or in the presence of an excess of the reagent of formula (V), optionally in a reaction-inert solvent;

c) N-alkylating an intermediate of formula

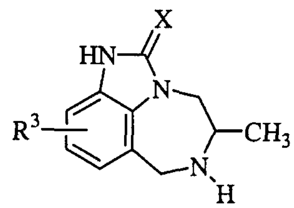

(VI)

wherein $R^3$and X are as defined in formula (I) with a reagent of formula $R^1R^2C=CH-Alk-W$ (VII) wherein W represents a reactive leaving group and $R^1$ and $R^2$ are as defined in formula (I), in a reaction-inert solvent;

d) cyclizing a benzimidazole of formula :

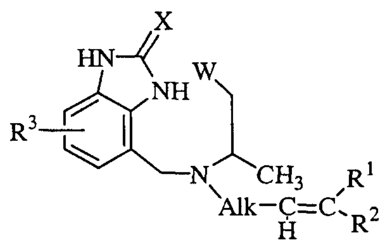

(XI)

wherein $R^1$, $R^2$, $R^3$, Alk and X are as defined in formula (I) and W represents a reactive leaving group, in a reaction-inert solvent, optionally in the presence of a base and optionally at an elevated temperature; and, if desired, converting the compounds of formula (I) into a therapeutically active non-toxic acid addition salt form by treatment with an acid; or conversely, converting the acid salt into the free base with alkali; and/or preparing stereochemically isomeric forms thereof.

**2.** A process according to claim 1 preparing a compound of formula (I) wherein X represents O.

**3.** A process according to claim 1 preparing a compound of formula (I) wherein X represents S.

**4.** A process according to claim 1 preparing a compound of formula (I) wherein the compound is (+)-(S)-8-chloro-6-(3-ethyl-2-pentenyl)-4,5,6,7-tetrahydro-5-methylimidazo[4,5,1-jk] [1,4]benzodiazepin-2(1H)-thione.

**5.** A method of preparing a pharmaceutical composition comprising a pharmaceutically acceptable carrier and as active ingredient an effective antiviral amount of a compound as described in any of claims 1-4, **characterized in that** a therapeutically effective amount of a compound as defined in any of claims 1 to 4 is intimately mixed with a pharmaceutical carrier.

**6.** A process of preparing a compound as defined in any of claims 1,3 or 4 and wherein X represents S, **characterized by**

a) thionating a compound of formula :

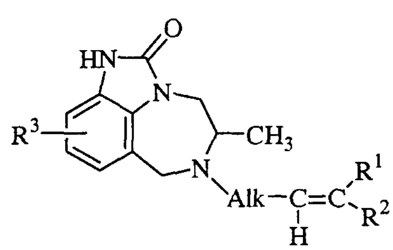

(I-b-1)

wherein $R^1$, $R^2$, $R^3$ and Alk are as defined in formula (I) with phosphorus pentasulfide in a reaction-inert solvent, thus yielding a compound of formula :

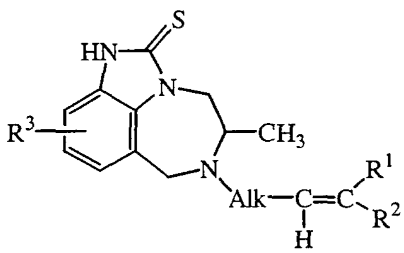

(I-b-2)

b) thiating a tetrahydroimidazo[4,5,1-jk][1,4]benzodiazepine of formula :

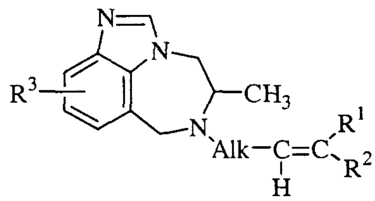

(IX)

wherein $R^1$, $R^2$, $R^3$ and Alk are as defined in formula (I) with elemental sulfur at an elevated temperature, thus yielding a compound of formula (I-b-2);

c)reducing and thiocarbonylating a 9-nitrobenzodiazepine of formula :

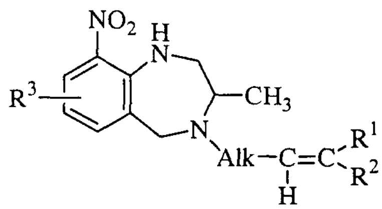

(X)

wherein $R^1$, $R^2$, $R^3$ and Alk are as defined in formula (I) in the presence of an alkali metal sulfide or hydrogen sulfide, and carbon disulfide, thus yielding a compound of formula (I-b-2); and if desired, converting the compounds of formula (I) into a therapeutically active non-toxic acid addition salt form by treatment with an acid; or conversely, converting the acid salt into the free base with alkali; and/or preparing stereochemically isomeric forms thereof.

**Patentansprüche**

**Patentansprüche für folgende Vertragsstaaten : AT, SE**

**1.** Verbindungen der Formel:

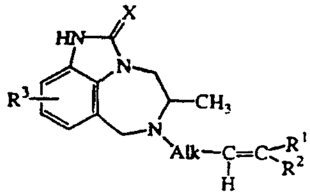

(I),

deren pharmazeutisch unbedenkliche Säureadditionssalze und deren stereochemisch isomere Formen, wobei

X für O oder S steht;

Alk für $C_{1-6}$Alkandiyl steht;
$R^1$ und $R^2$ für $C_{1-4}$Alkyl stehen;
$R^3$ für Methyl oder Chlor steht.

**2.** Verbindungen nach Anspruch 1, wobei X für O steht.

**3.** Verbindungen nach Anspruch 1, wobei X für S steht.

**4.** Verbindungen nach Anspruch 1, bei der es sich um (+)-(S)-8-Chlor-6-(3-ethyl-2-pentenyl)-4,5,6,7-tetrahydro-5-methylimidazo[4,5,1-jk][1,4]benzodiazepin-2(1H)-thion handelt.

**5.** Antivirale Zusammensetzung, enthaltend einen pharmazeutisch unbedenklichen Trägerstoff und als Wirkstoff eine antiviral wirksame Menge einer Verbindung nach einem der Ansprüche 1-4.

**6.** Verfahren zur Herstellung einer pharmazeutischen Zusammensetzung nach Anspruch 5, **dadurch gekennzeichnet, daß** man eine therapeutisch wirksame Menge einer Verbindung nach einem der Ansprüche 1 bis 4 innig mit einem pharmazeutischen Trägerstoff mischt.

**7.** Verbindungen nach einem der Ansprüche 1 bis 4 zur Verwendung als Arzneimittel.

**8.** Verfahren zur Herstellung einer Verbindung nach einem der Ansprüche 1 bis 4,
**dadurch gekennzeichnet, daß** man

a) ein 9-Amino-2,3,4,5-tetrahydro-lH-1,4-benzodiazepin der Formel:

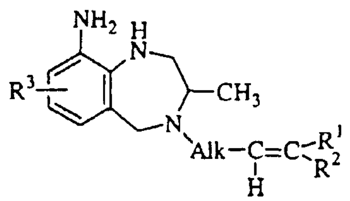

(II),

wobei $R^1$, $R^2$, $R^3$ und Alk wie unter Formel (I) definiert sind, mit einem Reagens der Formel L-C(=X)-L (III), wobei L für eine reaktive Abgangsgruppe und X für O oder S steht, in einem reaktionsinerten Lösungsmittel kondensiert;
b) ein 4,5,6,7-Tetrahydroimidazo[4,5,1-jk][1,4]benzodiazepin der Formel:

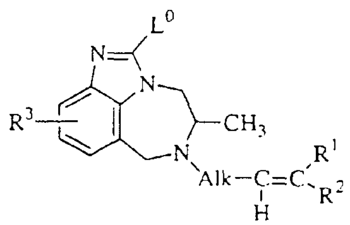

(IV)

wobei $R^1$, $R^2$, $R^3$ und Alk wie unter Formel (I) definiert sind und $L^0$ für eine reaktive Abgangsgruppe steht, mit einem Reagens der Formel $M_2$-X (V), wobei X wie unter Formel (I) definiert ist, in einem reaktionsinerten Lösungsmittel oder in Gegenwart eines Überschusses des Reagens der Formel (V) und gegebenenfalls in einem reaktionsinerten Lösungsmittel umsetzt;
c) ein Zwischenprodukt der Formel

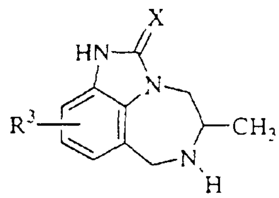

(VI)

wobei $R^3$ und X wie unter Formel (I) definiert sind, mit einem Reagens der Formel $R^1R^2C=CH$-Alk-W (VII), wobei W für eine reaktive Abgangsgruppe steht und $R^1$ und $R^2$ wie unter Formel (I) definiert sind, in einem reaktionsinerten Lösungsmittel N-alkyliert;

d) ein Benzimidazol der Formel:

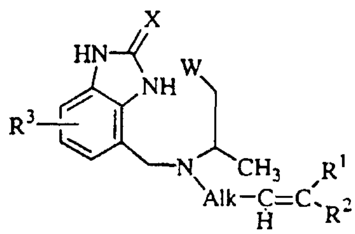

(XI)

wobei $R^1$, $R^2$, $R^3$, Alk und X wie unter Formel (I) definiert sind und W für eine reaktive Abgangsgruppe steht, in einem reaktionsinerten Lösungsmittel, gegebenenfalls in Gegenwart einer Base und gegebenenfalls bei erhöhter Temperatur, zyklisiert; und gewünschtenfalls die Verbindungen der Formel (I) durch Behandlung mit einer Säure in eine therapeutisch wirksame nichttoxische Säureadditionssalzform umwandelt; oder umgekehrt das Säuresalz mit Alkali in die freie Base umwandelt; und/oder stereochemisch isomere Formen davon herstellt.

**9.** Verfahren zur Herstellung einer Verbindung nach einem der Ansprüche 1, 3 oder 4, wobei X für S steht, **dadurch gekennzeichnet, daß** man

a) eine Verbindung der Formel

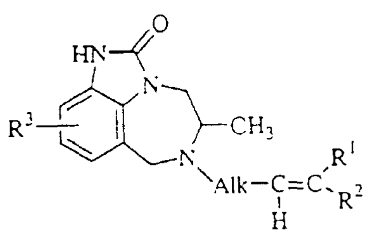

(I-b-1)

wobei $R^1$, $R^2$, $R^3$ und Alk wie unter Formel (I) definiert sind, mit Phosphorpentasulfid in einem reaktionsinerten Lösungsmittel thioniert und so eine Verbindung der Formel:

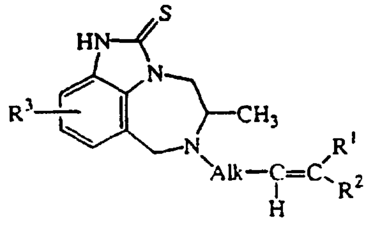

(I-b-2)

erhält;

b) ein Tetrahydroimidazo[4,5,1-jk][1,4]benzodiazepin der Formel:

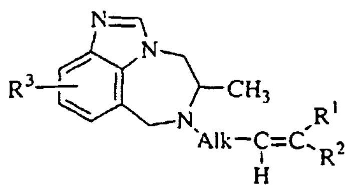

(IX)

wobei $R^1$, $R^2$, $R^3$ und Alk wie unter Formel (I) definiert sind, mit elementarem Schwefel bei erhöhter Temperatur thiiert und so eine Verbindung der Formel (I-b-2) erhält;

c) ein 9-Nitrobenzodiazepin der Formel:

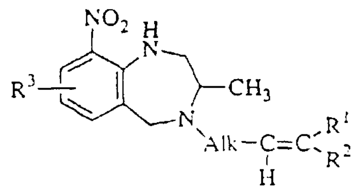

(X)

wobei $R^1$, $R^2$, $R^3$ und Alk wie unter Formel (I) definiert sind, in Gegenwart eines Alkalisulfids oder von Schwefelwasserstoff, und von Schwefelkohlenstoff, reduziert und thiocarbonyliert und so eine Verbindung der Formel (I-b-2) erhält; und gewünschtenfalls die Verbindungen der Formel (I) durch Behandlung mit einer Säure in eine therapeutisch wirksame nichttoxische Säureadditionssalzform umwandelt; oder umgekehrt das Säuresalz mit Alkali in die freie Base umwandelt; und/oder stereochemisch isomere Formen davon herstellt.

**Patentansprüche für folgende Vertragsstaaten : ES, GR**

**1.** Verfahren zur Herstellung von Verbindungen der Formel:

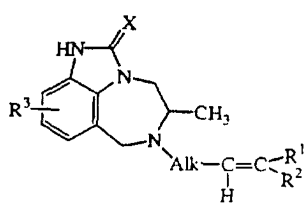

(I),

deren pharmazeutisch unbedenkliche Säureadditionssalze und deren stereochemisch isomere Formen, wobei

X für O oder S steht;
Alk für $C_{1-6}$Alkandiyl steht;
$R^1$ und $R^2$ für $C_{1-4}$Alkyl stehen;
$R^3$ für Methyl oder Chlor steht;

**dadurch gekennzeichnet, daß** man:

a) ein 9-Amino-2,3,4,5-tetrahydro-lH-1,4-benzodiazepin der Formel:

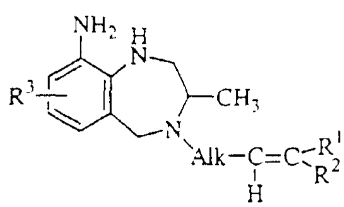

(II),

wobei $R^1$, $R^2$, $R^3$ und Alk wie unter Formel (I) definiert sind, mit einem Reagens der Formel L-C(=X)-L (III), wobei L für eine reaktive Abgangsgruppe und X für O oder S steht, in einem reaktionsinerten Lösungsmittel kondensiert;

b) ein 4,5,6,7-Tetrahydroimidazo[4,5,1-jk][1,4]benzodiazepin der Formel:

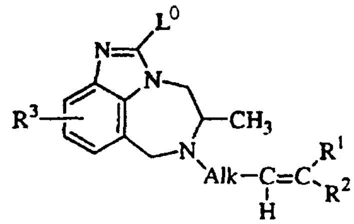

(IV)

wobei $R^1$, $R^2$, $R^3$ und Alk wie unter Formel (I) definiert sind und $L^0$ für eine reaktive Abgangsgruppe steht, mit einem Reagens der Formel $M_2$-X (V), wobei X wie unter Formel (I) definiert ist, in einem reaktionsinerten Lösungsmittel oder in Gegenwart eines Überschusses des Reagens der Formel (V) und gegebenenfalls in einem reaktionsinerten Lösungsmittel umsetzt;

c) ein Zwischenprodukt der Formel

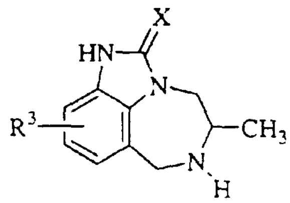

(VI)

wobei $R^3$ und X wie unter Formel (I) definiert sind, mit einem Reagens der Formel $R^1R^2C$=CH-Alk-W (VII), wobei W für eine reaktive Abgangsgruppe steht und $R^1$ und $R^2$ wie unter Formel (I) definiert sind, in einem reaktionsinerten Lösungsmittel N-alkyliert;

d) ein Benzimidazol der Formel:

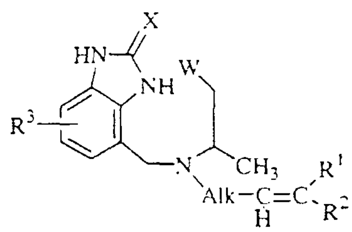

(XI)

wobei $R^1$, $R^2$, $R^3$, Alk und X wie unter Formel (I) definiert sind und W für eine reaktive Abgangsgruppe steht, in einem reaktionsinerten Lösungsmittel, gegebenenfalls in Gegenwart einer Base und gegebenenfalls bei erhöhter Temperatur, zyklisiert; und gewünschtenfalls die Verbindungen der Formel (I) durch Behandlung mit einer Säure in eine therapeutisch wirksame nichttoxische Säureadditionssalzform umwandelt; oder umgekehrt das Säuresalz mit Alkali in die freie Base umwandelt; und/oder stereochemisch isomere Formen davon herstellt.

**2.** Verfahren nach Anspruch 1 zur Herstellung einer Verbindung der Formel (I), in der X für O steht.

**3.** Verfahren nach Anspruch 1 zur Herstellung einer Verbindung der Formel (I), in der X für S steht.

**4.** Verfahren nach Anspruch 1 zur Herstellung einer Verbindung der Formel (I), bei der es sich um (+)-(S)-8-Chlor-6-(3-ethyl-2-pentenyl)-4,5,6,7-tetrahydro-5-methylimidazo[4,5,1-jk] [1,4]benzodiazepin-2(1H)-thion handelt.

**5.** Verfahren zur Herstellung einer pharmazeutischen Zusammensetzung enthaltend einen pharmazeutisch unbedenklichen Trägerstoff und als Wirkstoff eine antiviral wirksame Menge einer Verbindung nach einem der Ansprüche 1-4, **dadurch gekennzeichnet, daß** man eine therapeutisch wirksame Menge einer Verbindung nach einem der Ansprüche 1 bis 4 innig mit einem pharmazeutischen Trägerstoff mischt.

**6.** Verfahren zur Herstellung einer Verbindung nach einem der Ansprüche 1, 3 oder 4, wobei X für S steht, **dadurch gekennzeichnet, daß** man

a) eine Verbindung der Formel

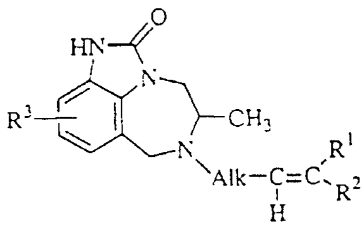

(I-b-1)

wobei $R^1$, $R^2$, $R^3$ und Alk wie unter Formel (I) definiert sind, mit Phosphorpentasulfid in einem reaktionsinerten Lösungsmittel thioniert und so eine Verbindung der Formel:

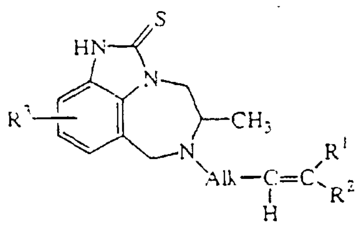

(I-b-2)

erhält;
b) ein Tetrahydroimidazo[4,5,1-jk][1,4]benzodiazepin der Formel:

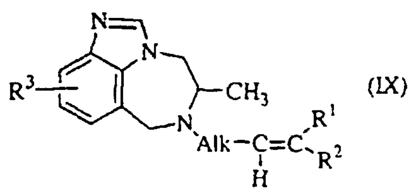

(IX)

wobei $R^1$, $R^2$, $R^3$ und Alk wie unter Formel (I) definiert sind, mit elementarem Schwefel bei erhöhter Temperatur thiiert und so eine Verbindung der Formel (I-b-2) erhält;
c) ein 9-Nitrobenzodiazepin der Formel:

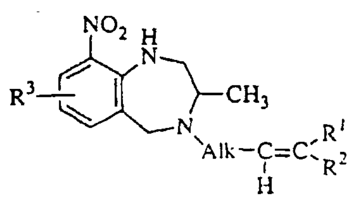

(X)

wobei $R^1$, $R^2$, $R^3$ und Alk wie unter Formel (I) definiert sind, in Gegenwart eines Alkalisulfids oder von Schwefelwasserstoff, und von Schwefelkohlenstoff, reduziert und thiocarbonyliert und so eine Verbindung der Formel (I-b-2) erhält; und gewünschtenfalls die Verbindungen der Formel (I) durch Behandlung mit einer Säure in eine therapeutisch wirksame nichttoxische Säureadditionssalzform umwandelt; oder umgekehrt das Säuresalz mit Alkali in die freie Base umwandelt; und/oder stereochemisch isomere Formen davon herstellt.

## Revendications

### Revendications pour les Etats contractants suivants : AT, SE

**1.** Composé répondant à la formule:

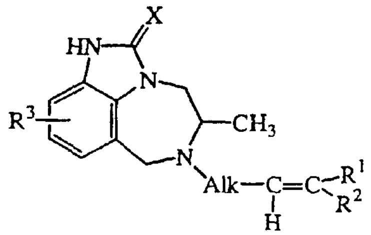

(I),

sel d'addition d'acide pharmaceutiquement acceptable ou forme stéréoisomère de ce composé, dans lequel

X est O ou S;
Alk est un groupe alcanediyle en $C_1$-$C_6$;
$R^1$ et $R^2$ sont des groupes alkyle en $C_{1-4}$;
$R^3$ est un groupe méthyle ou chloro.

**2.** Composé selon la revendication 1, dans lequel X représente O.

**3.** Composé selon la revendication 1, dans lequel X représente S.

**4.** Composé selon la revendication 1, dans lequel le composé est 1a (+)-(S)-8-chloro-6-(3-éthyl-2-pentényl)-4,5,6,7-tétrahydro-5-méthylimidazo[4,5,1-jk]-[1,4]benzodiazépine-2(1H)-thione.

**5.** Composition antivirale comprenant un véhicule pharmaceutiquement acceptable et, comme principe actif, une quantité antivirale efficace d'un composé selon l'une quelconque des revendications 1-4.

**6.** Procédé de préparation d'une composition pharmaceutique selon la revendication 5, **caractérisé en ce qu'**une quantité efficace sur le plan thérapeutique d'un composé selon l'une quelconque des revendications 1 à 4 est intimement mélangée avec un véhicule pharmaceutique.

**7.** Composé selon l'une quelconque des revendications 1 à 4 à utiliser comme médicament.

**8.** Procédé de préparation d'un composé selon l'une quelconque des revendications 1 à 4, **caractérisé par** les étapes suivantes:

a) condensation d'une 9-amino-2,3,4,5-tétrahydro-1H-1,4-benzodiazépine de formule:

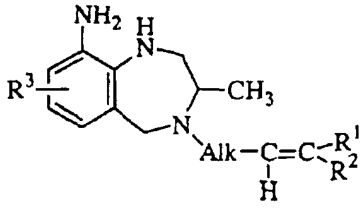

(II),

dans laquelle $R^1$, $R^2$, $R^3$ et Alk sont tels que définis dans la formule (I), avec un réactif de formule L-C(=X)-L (III), dans laquelle L est un groupe partant réactif et X est O ou S, dans un solvant inerte vis-à-vis de la réaction;

b) réaction d'une 4,5,6,7-tétrahydroimidazo[4,5,1-jk][1,4]benzodiazépine de formule:

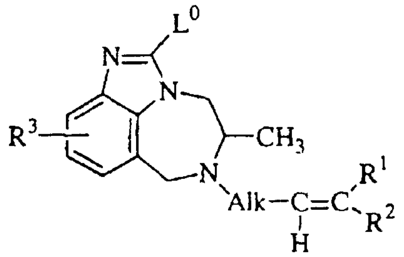

(IV)

dans laquelle $R^1$, $R^2$, $R^3$ et Alk sont tels que définis dans la formule (I) et L° est un groupe partant réactif, avec un réactif de formule $M_2$-X (V), dans laquelle X est tel que défini dans la formule (I), dans un solvant inerte vis-à-vis de la réaction ou en présence d'un excès du réactif de formule (V), éventuellement dans un solvant inerte vis-à-vis de la réaction;

c) N-alkylation d'un intermédiaire de formule

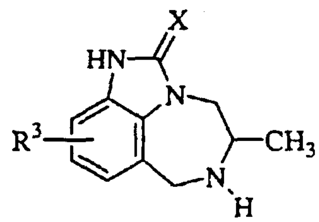

(VI)

dans laquelle $R^3$ et X sont tels que définis dans la formule (I), avec un réactif de formule $R^1R^2C$=CH-Alk-W (VII), dans laquelle W représente un groupe partant réactif et $R^1$ et $R^2$ sont tels que définis dans la formule (I), dans un solvant inerte vis-à-vis de la réaction;

d) cyclisation d'un benzimidazole de formule:

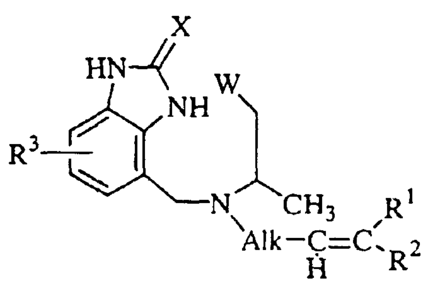

(XI)

dans laquelle $R^1$, $R^2$, $R^3$, Alk et X sont tels que définis dans la formule (I) et W représente un groupe partant réactif, dans un solvant inerte vis-à-vis de la réaction, éventuellement en présence d'une base et éventuellement à une température élevée; et, le cas échéant, conversion des composés de formule (I) en un sel d'addition d'acide non toxique, actif sur le plan thérapeutique, par traitement avec un acide; ou inversement, conversion du sel d'acide en base libre avec une base; et/ou préparation de formes stéréoisomères de ces composés.

**9.** Procédé de préparation d'un composé selon l'une quelconque des revendications 1, 3 ou 4 et dans lequel X représente S, **caractérisé par** les étapes suivantes:

a) thionation d'un composé de formule:

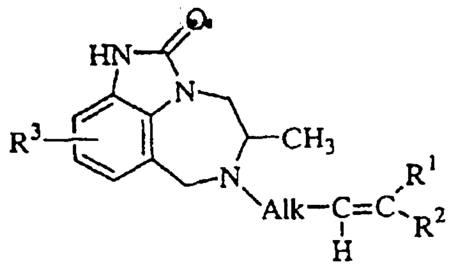

(I-b-1)

dans laquelle $R^1$, $R^2$, $R^3$ et Alk sont tels que définis dans la formule (I), avec du pentasulfure de phosphore, dans un solvant inerte vis-à-vis de la réaction, pour donner un composé de formule:

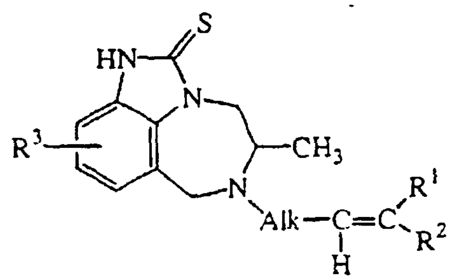

(I-b-2)

b) thiation d'une tétrahydroimidazo[4,5,1-jk]-[1,4]benzodiazépine de formule:

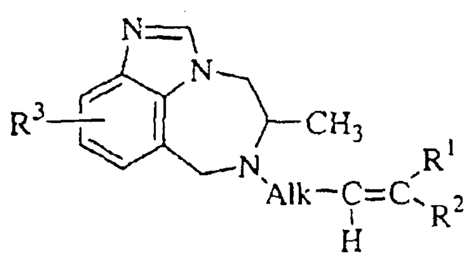

(IX)

dans laquelle $R^1$, $R^2$, $R^3$ et Alk sont tels que définis dans la formule (I), avec du soufre élémentaire, à une température élevée, pour donner un composé de formule (I-b-2);
c) réduction et thiocarbonylation d'une 9-nitrobenzodiazépine de formule:

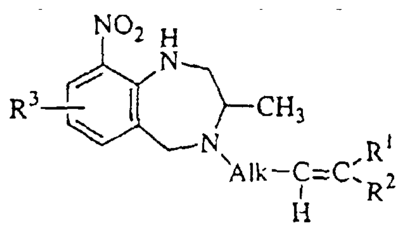

(X)

dans laquelle $R^1$, $R^2$, $R^3$ et Alk sont tels que définis dans la formule (I), en présence d'un sulfure de métal alcalin ou de sulfure d'hydrogène, et de disulfure de carbone, pour donner un composé de formule (I-b-2); et, le cas échéant, conversion des composés de formule (I) en sel d'addition d'acide non toxique, actif sur le plan thérapeutique, par traitement avec un acide; ou inversement, conversion du sel d'acide en base libre avec une base; et/ou préparation de formes stéréoisomères de ces composés.

**Revendications pour les Etats contractants suivants : ES, GR**

**1.** Procédé de préparation d'un composé répondant à la formule:

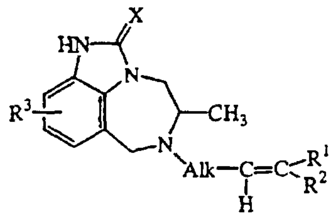

(I),

d'un sel d'addition d'acide pharmaceutiquement acceptable ou d'une forme stéréoisomère de ce composé, dans lequel

X est O ou S;
Alk est un groupe alcanediyle en $C_1$-$C_6$;
$R^1$ et $R^2$ sont des groupes alkyle en $C_{1-4}$;
$R^3$ est un groupe méthyle ou chloro;

**caractérisé par** les étapes suivantes:

a) condensation d'une 9-amino-2,3,4,5-tétrahydro-1H-1,4-benzodiazépine de formule:

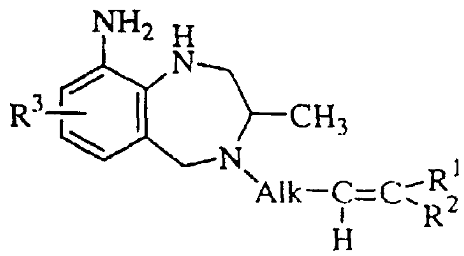

(II),

dans laquelle $R^1$, $R^2$, $R^3$ et Alk sont tels que définis dans la formule (I), avec un réactif de formule L-C(=X)-L (III), dans laquelle L est un groupe partant réactif et X est O ou S, dans un solvant inerte vis-à-vis de la réaction;
b) réaction d'une 4,5,6,7-tétrahydroimidazo[4,5,1-jk][1,4]benzodiazépine de formule:

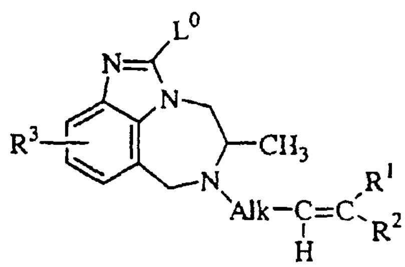

(IV)

dans laquelle $R^1$, $R^2$, $R^3$ et Alk sont tels que définis dans la formule (I) et $L^0$ est un groupe partant réactif, avec un réactif de formule $M_2$-X (V), dans laquelle X est tel que défini dans la formule (I), dans un solvant inerte vis-à-vis de la réaction ou en présence d'un excès du réactif de formule (V), éventuellement dans un solvant inerte vis-à-vis de la réaction;
c) N-alkylation d'un intermédiaire de formule

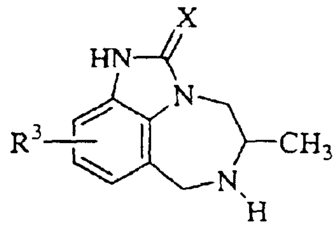

(VI)

dans laquelle $R^3$ et X sont tels que définis dans la formule (I), avec un réactif de formule $R^1R^2C=CH\text{-}Alk\text{-}W$ (VII), dans laquelle W représente un groupe partant réactif et $R^1$ et $R^2$ sont tels que définis dans la formule (I), dans un solvant inerte vis-à-vis de la réaction;

d) cyclisation d'un benzimidazole de formule:

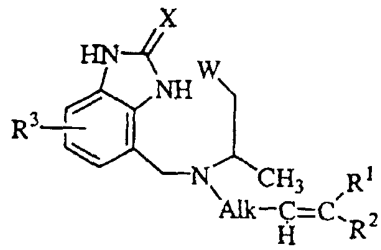

(XI)

dans laquelle $R^1$, $R^2$, $R^3$, Alk et X sont tels que définis dans la formule (I) et W représente un groupe partant réactif, dans un solvant inerte vis-à-vis de la réaction, éventuellement en présence d'une base et éventuellement à une température élevée; et, le cas échéant, conversion des composés de formule (I) en un sel d'addition d'acide non toxique, actif sur le plan thérapeutique, par traitement avec un acide; ou inversement, conversion du sel d'acide en base libre avec une base; et/ou préparation de formes stéréoisomères de ces composés.

**2.** Procédé selon la revendication 1 de préparation d'un composé de formule (I) dans laquelle X représente O.

**3.** Procédé selon la revendication 1 de préparation d'un composé de formule (I) dans laquelle X représente S.

**4.** Procédé selon la revendication 1 de préparation d'un composé de formule (I) dans lequel le composé est la (+)-(S)-8-chloro-6-(3-éthyl-2-pentényl)-4,5,6,7-tétrahydro-5-méthylimidazo[4,5,1-jk][1,4]benzodiazépine-2(1H)-thione.

**5.** Procédé de préparation d'une composition pharmaceutique comprenant un véhicule pharmaceutiquement acceptable et, comme principe actif, une quantité antivirale efficace d'un composé tel que décrit dans l'une quelconque des revendications 1-4, **caractérisé en ce que** la quantité efficace sur le plan thérapeutique d'un composé tel que défini dans l'une quelconque des revendications 1 à 4 est intimement mélangée avec un véhicule pharmaceutique.

**6.** Procédé de préparation d'un composé tel que défini dans l'une quelconque des revendications 1, 3 ou 4, et dans lequel X représente S, **caractérisé par** les étapes suivantes:

a) thionation d'un composé de formule:

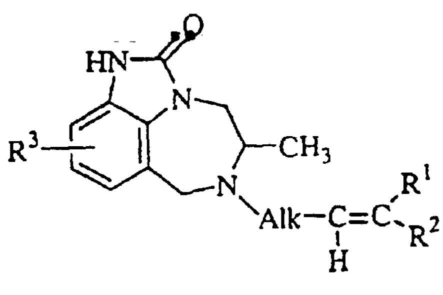

(I-b-1)

dans laquelle $R^1$, $R^2$, $R^3$ et Alk sont tels que définis dans la formule (I), avec du pentasulfure de phosphore, dans un solvant inerte vis-à-vis de la réaction, pour donner un composé de formule:

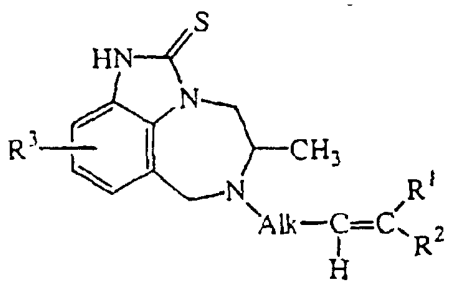

(I-b-2)

b) thiation d'une tétrahydroimidazo[4,5,1-jk]-[1,4]benzodiazépine de formule:

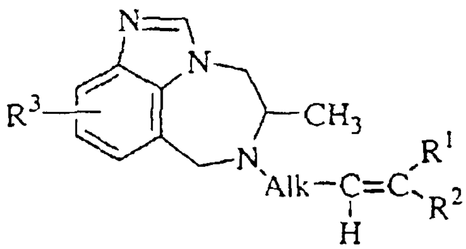

(IX)

dans laquelle $R^1$, $R^2$, $R^3$ et Alk sont tels que définis dans la formule (I), avec du soufre élémentaire, à une température élevée, pour donner un composé de formule (I-b-2) ;
c) réduction et thiocarbonylation d'une 9-nitrobenzodiazépine de formule:

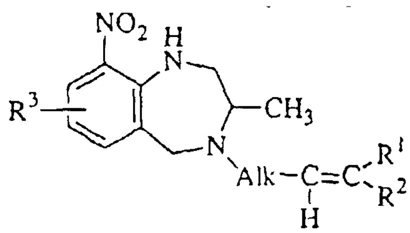

(X)

dans laquelle $R^1$, $R^2$, $R^3$ et Alk sont tels que définis dans la formule (I), en présence d'un sulfure de métal alcalin ou de sulfure d'hydrogène, et de disulfure de carbone, pour donner un composé de formule (I-b-2); et, le cas échéant, conversion des composés de formule (I) en un sel d'addition d'acide non toxique, actif sur le plan thérapeutique, par traitement avec un acide; ou inversement, conversion du sel d'acide en base libre avec une base; et/ou préparation de formes stéréoisomères de ces composés.